# EUROPEAN PATENT SPECIFICATION

(11) **EP 4 306 161 B1**
(45) Date of publication and mention of the grant of the patent: **07.05.2025**
(21) Application number: 22184564.7
(22) Date of filing: 12.07.2022
(51) Int. Cl.: A61N 1/05, A61N 1/32, A61N 1/36

(54) **DEVICE FOR STIMULATING A NERVE OR A PART OF A NERVOUS SYSTEM**
VORRICHTUNG ZUM STIMULIEREN EINES NERVS ODER EINES TEILS EINES NERVENSYSTEMS
DISPOSITIF DE STIMULATION DE NERF OU D'UNE PARTIE D'UN SYSTÈME NERVEUX

(43) Date of publication of application: 17.01.2024
(73) Proprietor: Stichting IMEC Nederland, 5656 AE Eindhoven (NL)
(72) Inventor: MIHAJLOVIC, Vojkan, 5643 AZ Eindhoven (NL); FICHMAN, Mark, 5654 KC Eindhoven (NL)
(74) Representative: AWA Sweden AB

(56) References cited:
- WO-A1-2013/043267
- US-A1- 2011 295 339
- US-A1- 2018 193 640
- US-A1- 2020 324 119
- US-A1- 2021 138 245
- JABBAN LEEN ET AL: "Interferential Current Stimulation for Non-Invasive Somatotopic Sensory Feedback for Upper-Limb Prosthesis: Simulation Results using a Computable Human Phantom", 2021 10TH INTERNATIONAL IEEE/EMBS CONFERENCE ON NEURAL ENGINEERING (NER), IEEE, 4 May 2021 (2021-05-04), pages 765 - 768, XP033920507, DOI: 10.1109/NER49283.2021.9441299

## Description

### Technical field

The present description relates to stimulation of a nerve or a part of a nervous system of a living being. In particular, the present description relates to a device for stimulating a nerve or a part of a nervous system and to a method for controlling stimulation.

### Background

Neurostimulation may be used for controlling a function of an organ or part of a body of a living being, such as a human being, e.g., for alleviating or treating a number of conditions of the human being. Neurostimulation may involve a control of electrical signals transported through a nerve or a part of a nervous system such as a ganglion, so as to trigger or block signals transported by the nerve or the ganglion.

Stimulation of a target may be provided using a plurality of electrodes arranged in different locations in relation to the target. In particular, if it is desired to target a specific part of the nerve, such as a fascicle or nerve fiber(s) within the fascicle, use of the plurality of electrodes is advantageous. Typically, a signal provided to a nerve will attenuate with a depth into the nerve such that a maximum electric field is provided at an outer part of the nerve, in epineurium. In particular, since the epineurium has low conductive properties, large signals are required for reaching deep into the nerve. However, signals from two pairs of electrodes may be provided so as to interfere within the nerve and selectively provide a large electric field within the nerve, forming interferential stimulation of the nerve. This implies that a part of the nerve inside the epineurium may be stimulated while using modest signal levels.

However, there is still a need to improve interferential stimulation to allow accurate stimulation of a target such that only desired portion(s) of a nerve or a part of a nervous system are activated by the stimulation.

US 2021/0138245 discloses a method for spinal cord stimulation treatment that includes positioning eight implantable electrodes to a dura matter in an epidural space proximate to a subject's spinal cord so that (i) a first circuit is created between a first and second electrode on a first channel, (ii) a second circuit is created between a third and fourth electrode on a second channel, (iii) a third circuit is created between a fifth and sixth electrode on a third channel, and (iv) a fourth circuit is created between a seventh and eighth electrode on a fourth channel, transmitting signals through the first and second circuits that interfere to produce a first beat signal, transmitting signals through the third and fourth circuits that interfere to produce a second beat signal, and interaction of the first and second beat signals results in a combined beat signal proximate to and/or within the subject's spinal cord.

US 2020/0324119 discloses that a system for delivering neurostimulation energy may include a programming control circuit and a user interface. The programming control circuit may be configured to generate stimulation parameters according to a neurostimulation program including a pattern of interferential stimulation configured to effect asynchronous and/or non-regular activation of nerve fibers by simultaneously delivering a first stimulation current having a first waveform with a first frequency using a first electrode configuration and a second stimulation current having a second waveform with a second frequency using a second electrode configuration. The user interface may be configured to determine the neurostimulation program and to provide the pattern of interferential stimulation with modulation of the first waveform, the second waveform, the first electrode configuration, and/or the second electrode configuration to result in a time-varying beat frequency capable of effecting the asynchronous and/or non-regular activation of the nerve fibers.

### Summary

An objective of the present description is to provide an accurate control of interferential stimulation of a nerve or a part of a nervous system.

This and other objectives are at least partly met by the invention as defined in the independent claims. Preferred embodiments are set out in the dependent claims.

According to a first aspect, there is provided a device for stimulating a nerve or a part of a nervous system of a living being, said device comprising: a stimulation generating unit configured to generate stimulation signals for stimulating the nerve or the part of the nervous system; a first pair of electrodes configured to be arranged in a first location in relation to the nerve or the part of the nervous system; a second pair of electrodes configured to be arranged in a second location in relation to the nerve or the part of the nervous system; a third pair of electrodes configured to be arranged in a third location in relation to the nerve or the part of the nervous system; wherein the stimulation generating unit is configured to generate a first waveform comprising a first frequency to the first pair of electrodes; a second waveform comprising a second frequency to the second pair of electrodes, and a third waveform comprising a third frequency to the third pair of electrodes; wherein the first frequency and the second frequency define a beat frequency corresponding to a difference between the first frequency and the second frequency and wherein the third frequency is related to the beat frequency by an integer number, wherein the stimulation generating unit is configured to output the first waveform, the second waveform, and the third waveform for causing interferential stimulation in the nerve or the part of the nervous system.

Thanks to the device being configured to generate the first waveform comprising the first frequency to the first pair of electrodes and the second waveform comprising the second frequency to the second pair of electrodes, the device is able to define an interference between the first waveform and the second waveform in the nerve or the part of the nervous system. When the first waveform and the second waveform interfere, the interference may form a varying signal having a frequency corresponding to the difference in frequency between the first frequency and the second frequency. This frequency of the interference between the first and second waveforms may be referred to as a beat frequency.

Thanks to the device being further configured to generate the third waveform comprising a third frequency to the third pair of electrodes, wherein the third frequency is related to the beat frequency by an integer number, the third waveform may constructively interfere with the first and second waveforms such that a stimulation signal in a particular portion of the nerve or the part of the nervous system may be amplified and cause interferential stimulation. The third waveform thus allows accurately selecting a location in the nerve or the part of the nervous system in which interferential stimulation will occur.

The first frequency and the second frequency may be selected to be high frequencies, whereas the beat frequency represents a low frequency lower than the first frequency and the second frequency. The nerve or the part of the nervous system may be affected to a larger extent by low frequency signals than high frequency signals. Thus, the first waveform and the second waveform may include a first frequency and a second frequency, respectively, which do not affect, or minimally affect, the nerve or the part of the nervous system. The first waveform and the second waveform may act to block a signal transported by the nerve or the part of the nervous system. Further, an amplitude of the first waveform and an amplitude of the second waveform may be smaller than an amplitude of an interferential signal with the beat frequency, such that a sufficiently large signal (amplitude) for stimulating the nerve is only provided by the interferential signal.

The third waveform modulates a beat frequency signal defined by interference of the first waveform and the second waveform such that an amplitude of the beat frequency signal is substantially increased. In particular, thanks to the third frequency being related to the beat frequency by an integer number, the third waveform may be matched to the beat frequency signal in order to amplify the beat frequency signal and to control stimulation of the nerve or the part of the nervous system. A phase of the third waveform may be controlled in relation to a phase of the beat frequency signal such that constructive interference may be achieved so as to cause interferential stimulation in the nerve or the part of the nervous system. Also, modulation of the beat frequency by the third waveform enables only positive current flow to be provided during a first half cycle of stimulation, followed by only negative current flow during a second half cycle of stimulation. This is similar to physiological principles of action potential generation and thus facilitates efficient stimulation of the nerve or part of the nervous system.

The first, second and third waveforms may also or alternatively be controlled to minimize an amplitude of the interferential signal at a desired location in the nerve or the part of the nervous system to selectively provide quiescent activation of the nerve or the part of the nervous system in the desired location, whereas the first, second and third waveforms may provide high activation in other locations or regions of the nerve or the part of the nervous system. Hence, high activation of a large region of the nerve or the part of the nervous system may be provided while quiescent activation is provided in a selected desired location.

Thanks to the use of the first, second, and third pair of electrodes for causing interferential stimulation, the electrodes may be arranged externally to the nerve or the part of the nervous system while providing stimulation in a desired location within the nerve or the part of the nervous system.

For instance, the electrodes may be arranged at a surface of a nerve. The interferential stimulation may be selectively achieved in a desired location within the nerve without causing stimulation in locations close to the surface and the electrodes. Using interference between the first, second, and third waveforms, the device allows accurate selection of a location in the nerve or the part of the nervous system to be stimulated.

The device may be used for stimulating a nerve of a living being. It should be realized that the device may be used for stimulating a nerve of a human being but that the device may likewise be used for stimulating nerves of animals.

As used herein, the term "nerve" should be construed as a bundle of nerve fibers enclosed by a sheath called the epineurium. The nerve may form part of the peripheral nervous system and may therefore also be called a peripheral nerve. The nerve is configured to transmit electrical impulses within the body along axons, which may be bundled in fascicles in fascicles in the nerve. The electrical impulses may be provided as action potentials transmitted along the axons.

The nerve may comprise a plurality of fascicles. Within the nerve, a large number of fascicles may be provided, and each fascicle may include a large number of axons, such that the nerve may transmit signals to / from various parts of the body. For instance, the vagus nerve is a cranial nerve providing signals between the brain and parts of the body. The vagus nerve may be relatively easy to access and a device for stimulating a nerve that is to be implanted in the human being may therefore be advantageously arranged in relation to the vagus nerve for providing stimulation of the vagus nerve. However, since the vagus nerve provides signals to many different parts of the body, it is also important that stimulation of the nerve is controlled through interferential stimulation to selectively stimulate a portion of the nerve for providing a desired effect of the stimulation.

Stimulation of the nerve may correspond to providing a sufficiently large action potential so as to trigger a signal to be transported by the nerve or block a signal from being transported by the nerve.

The device may be configured to stimulate a part of the nervous system which is not necessarily a nerve. According to an embodiment, the device may be configured to stimulate a part of the peripheral nervous system. For instance, the device may be configured to stimulate a ganglion in the peripheral nervous system.

Reference is made herein to "first", "second", and "third", such as "first pair of electrodes", "second pair of electrodes", and "third pair of electrodes". It should be realized that items referred to as "first" are not the same item as items referred to as "second" or "third" and items referred to as "second" are not the same as items referred to as "third". Thus, the first pair of electrodes, the second pair of electrodes and the third pair of electrodes are different from each other. The first location in relation to the nerve, the second location in relation to the nerve and the third location in relation to the nerve are different from each other. The first waveform, the second waveform and the third waveform are different from each other.

The stimulation generating unit is configured to generate current waveforms for providing a current to the nerve or the part of the nervous system. The current can be converted to a voltage within the nerve or the part of the nervous system based on transconductance of tissue such that an action potential for stimulating the nerve or the part of the nervous system is formed.

The stimulation generating unit may comprise a plurality of stimulation generating modules with separate current generators, each dedicated to generating the first waveform, the second waveform and the third waveform, respectively. This implies that the first waveform to be output to the first pair of electrodes, the second waveform to be output to the second pair of electrodes, and the third waveform to be output to the third pair of electrodes may be separately controlled to ensure that desired waveforms are output and that a waveform is not affected by generation of other waveforms in the stimulation generating unit. Hence, the interferential stimulation may be accurately controlled by separately controlling the first waveform, the second waveform, and the third waveform.

According to an embodiment, the device is configured to be at least partially implanted in the living being with the first pair of electrodes, the second pair of electrodes, and the third pair of electrodes arranged at different locations in relation to the nerve or the part of the nervous system.

Thus, the device is adapted to provide the pairs of electrodes close to or in contact with the nerve or the part of the nervous system to be stimulated. This implies that stimulation signals may be output by the device directly into the nerve or the part of the nervous system providing an accurate control of the stimulation within the nerve or the part of the nervous system.

The device may be configured to be completely implanted within the body of the living being. This facilitates use of the implanted device for providing stimulation of the nerve to the living being in a convenient way. In particular, when the device is implanted within the body of a human being, the stimulation of the nerve may be provided with minimal effect of everyday life of the human being for providing the stimulation.

The device may be configured to be partially implanted within the body of the living being. Thus, the first pair of electrodes and the second pair of electrodes may be implanted within the body. In some embodiments, the third pair of electrodes may be configured to be arranged externally to the body, e.g., mounted at the skin of the body, so as to provide the third waveform into the body to the nerve or the part of the nervous system. Thus, modulation of the interferential signal formed by the first and second waveforms may be provided from outside the body.

Implanted pairs of electrodes may be configured to receive stimulation signals from implanted parts of the stimulation generating unit. Thus, if only the first and second pairs of electrodes are implanted, stimulation generating modules for generating the first and second waveforms may also be implanted. This implies that there may be no need for wires connecting stimulation generating modules to pairs of electrodes through the skin of the living being.

Parts of device being implanted may be configured to communicate with parts of the device being arranged externally to the living being. If the device is completely implanted, the device may still be configured to communicate with an external unit. Communication between implanted parts and parts arranged externally to the living being may be achieved through wireless communication.

For instance, a fully implanted device may receive control signals from the external unit for changing parameters of the stimulation, such as parameters defining a stimulation paradigm, such as to control a location within the nerve to be stimulated or a frequency, an amplitude, or a duration of a stimulation to be provided.

The externally arranged device may be provided in a housing that may be worn by the living being or may be adapted for being arranged in vicinity of the living being. The implanted device or implanted parts of the device may be configured to communicate with a communication unit, which may be provided in a housing worn by the living being or arranged in vicinity of the living being, wherein the communication unit may act as an intermediate device for allowing the implanted device to communicate with an external unit which may be remotely arranged. Thus, communication between the communication unit and the external unit may be provided via a telecommunication or computer network.

The device may further comprise a control unit for controlling stimulation by the stimulation generating unit. It should further be realized that according to an embodiment, the control unit may be arranged in a separate housing from the stimulation generating unit. The control unit may then for instance be arranged in a housing that may be configured to be worn by the living being. The control unit may then be configured to provide control of the stimulation generating unit being implanted through wireless communication. However, the control unit may preferably be arranged in a common housing with the stimulation generating unit in order to ensure accurate control of the first waveform, the second waveform, and the third waveform.

Thus, the device may comprise a plurality of physical units which may or may not be connected by wires. If parts of the device are not physically connected, wireless communication may be used to allow the parts to communicate. For instance, if different stimulation generating modules are arranged in different housings, such as one stimulation generating module being arranged externally to the body of the living being for providing a third waveform to the third pair of electrodes being arranged external to the body and other stimulation generating modules being implanted for providing the first and the second waveforms to implanted first and second pairs of electrodes, respectively, wireless communication may be used for controlling timing of the first waveform, the second waveform, and the third waveform. For instance, the control unit may be arranged in one housing together with one or more stimulation generating modules and may communicate control signals wirelessly to other stimulation generating modules in other housings. Alternatively, the control unit may be distributed in plural control modules arranged in common housing with respective stimulation generating modules. The control modules may then communicate wirelessly for ensuring correct relative timing of the output of the first waveform, the second waveform, and the third waveform, respectively.

According to an embodiment, the electrodes of the first pair of electrodes and the second pair of electrodes are displaced in relation to each other along a longitudinal direction of the nerve. For instance, the electrodes of the first pair of electrodes may be arranged in different longitudinal positions in relation to the nerve. Also, the electrodes of the second pair of electrodes may be arranged in different longitudinal positions in relation to the nerve, which may be the same longitudinal positions as the longitudinal positions of the electrodes of the first pair of electrodes. The first waveform and the second waveform may thus each define electric fields along a longitudinal direction of the nerve extending into the nerve so as to form an interferential signal. The electrodes of the third pair of electrodes may also be arranged in different longitudinal positions in relation to the nerve, which may be the same longitudinal positions as the longitudinal positions of the electrodes of the first pair of electrodes and the electrodes of the second pair of electrodes. The third waveform may thus modulate the interferential signal to cause interferential simulation within a desired location of the nerve. Alternatively, the electrodes of the third pair of electrodes may be arranged at different positions along a circumference of the nerve at a longitudinal position between the longitudinal positions of the electrodes of the first and second pair of electrodes. Thus, the third waveform may be provided at a location where the interference between the first and second waveforms is strong.

According to another embodiment, the first pair of electrodes, the second pair of electrodes and the third pair of electrodes are arranged at different locations of a circumference of the nerve. Thus, the first waveform and the second waveform may mainly define electric fields based on the waveforms in different portions of a cross-section of the nerve. The first waveform and the second waveform may interfere within the cross-section of the nerve to define an interferential signal which may be modulated by the third waveform to cause stimulation of the nerve through interferential stimulation.

Both electrodes of the first pair of electrodes may be associated with a first part of the circumference of the nerve and both electrodes of the second pair of electrodes may be associated with a second part of the circumference of the nerve, wherein the second part is different from and non-overlapping with the first part. This implies that the portions of the cross-section of the nerve in which the first waveform and the second waveform, respectively, define electric fields are quite different so as to allow interferential stimulation deep within the nerve without necessarily providing stimulation in undesired locations of the nerve. In addition, each electrode of the third pair of electrodes may be arranged between an electrode of the first pair of electrodes and an electrode of the second pair of electrodes along the circumference of the nerve. For instance, the electrodes of the third pair of electrodes may be arranged on diametrically opposite sides of the nerve in order to enable the third waveform to interfere with the first and second waveforms in the desired location within the nerve.

Alternatively, the electrodes of the first pair of electrodes, the electrodes of the second pair of electrodes and the electrodes of the third pair of electrodes may be alternatingly arranged around the circumference of the nerve. This implies that the first waveform, the second waveform and the third waveform may define electric fields within the nerve with a large area of overlap of the electric fields based on the first waveform, the second waveform, and the third waveform, respectively. This may allow the location to be stimulated within the nerve to be selected more freely.

All of the electrodes of the first pair of electrodes, the second pair of electrodes and the third pair of electrodes may be arranged in relation to a common cross-section of the nerve. However, in other embodiments, the electrodes are arranged in relation to different cross-sections of the nerve while still being arranged such that the first waveform, the second waveform, and the third waveform interfere within the nerve.

Similarly, the electrodes of the first pair of electrodes, the second pair of electrodes and the third pair of electrodes may be arranged in relation to another part of the nervous system, which is not necessarily a nerve. For instance, the electrodes may be arranged in relation to a ganglion. Arrangement of the electrodes of the first pair of electrodes, the second pair of electrodes and the third pair of electrodes may be dependent on anatomy of the part of the nervous system. The electrodes may be suitably arranged externally to the part of the nervous system so as to define electric fields extending into the part of the nervous system so as to cause interferential stimulation in the part of the nervous system.

According to an embodiment, the device may comprise a set of electrodes, which may be arranged in a grid. The grid may be arranged on a surface of a common carrier, wherein the carrier may be flexible so as to allow the electrodes to be arranged in a contact with the part of the nervous system, such as in contact with a ganglion.

The device may be configured to select active electrodes among a set of electrodes to form the first pair of electrodes, the second pair of electrodes, and the third pair of electrodes.

According to an embodiment, the device comprises a carrier configured to be arranged in relation to the nerve or the part of the nervous system, wherein the first pair of electrodes, the second pair of electrodes, and the third pair of electrodes are mounted in or on the carrier.

Thus, the carrier may be arranged in relation to the nerve or the part of the nervous system, whereby the carrier will position the electrodes of the first pair of electrodes, the second pair of electrodes, and the third pair of electrodes in appropriate positions in relation to the nerve or the part of the nervous system. This facilitates implantation of the electrodes.

According to an embodiment, the device comprises a cuff configured to be arranged surrounding the circumference of the nerve, wherein the first pair of electrodes, the second pair of electrodes, and the third pair of electrodes are mounted in or on the cuff.

The cuff provides a convenient manner of arranging the electrodes in relation to a cross-section of the nerve. The cuff may be easily mounted around the nerve, whereby the cuff will position the electrodes of the first pair of electrodes, the second pair of electrodes, and the third pair of electrodes in appropriate positions in relation to the nerve. This facilitates implantation of the electrodes.

According to an embodiment, the beat frequency is two times larger than the third frequency.

This facilitates modulating the interference between the first and second waveforms so as to form an interferential signal with a similar signal shape as a typical monophasic or biphasic current stimulation which is realized in existing implants. Thus, a signal may be formed having two parts with opposite signs.

The beat frequency being two times larger than the third frequency may be particularly advantageous in forming a desired interferential signal in the nerve or the part of the nervous system. However, it should be realized that the third frequency may be related to the beat frequency by an integer number in other ways.

For instance, the third frequency may be equal to the beat frequency, or the third frequency may be two, three or four times larger than the beat frequency. This may still allow the third waveform to modulate the interference between the first and second waveforms so as to form an interferential signal that may efficiently cause stimulation in the nerve or the part of the nervous system.

According to an embodiment, the device further comprising a control unit configured to modulate the first waveform, the second waveform, and the third waveform by modulating the first frequency, the second frequency, and the third frequency, respectively, by modulating an amplitude of the first waveform, the second waveform, and the third waveform, respectively, and/or by modulating a time instant for start of the first waveform, the second waveform, and the third waveform, respectively, for controlling the interferential stimulation of the nerve or the part of the nervous system.

The control unit may be configured to control the stimulation generating unit for controlling the stimulation of the nerve or the part of the nervous system. The control unit may be configured to transmit control signals to the stimulation generating unit for controlling the stimulation generating unit and modulating the stimulation signals generated by the stimulation generating unit.

The control unit may be configured to control timing of output of the stimulation signals to the first pair of electrodes, the second pair of electrodes, and the third pair of electrodes. As mentioned above, the stimulation generating unit may comprise a plurality of stimulation generating modules with separate current generators, each dedicated to generating the first waveform, the second waveform and the third waveform, respectively. Thus, the control unit may be configured to provide synchronization of the stimulation generating modules to ensure that the first waveform, the second waveform, and the third waveform are output with a desired time relation.

The stimulation generating unit may comprise a plurality of stimulation generating modules with separate current generators. Thus, the stimulation generating unit may comprise a first current generator dedicated to generating the first waveform, a second current generator dedicated to generating the second waveform and a third current generator dedicated to generating the third waveform.

The first current generator, the second current generator, and the third current generator may each be configured to generate an alternating signal. The control unit may be configured to provide a control signal to the first current generator, the second current generator, and the third current generator, respectively, for modulating the alternating signal output by the first current generator, the second current generator, and the third current generator, respectively.

The control unit may be configured to modulate a first frequency of the alternating signal of the first waveform and/or to modulate a second frequency of the alternating signal of the second waveform. This implies that the beat frequency may be controlled, which may be used for controlling stimulation of the nerve. The control unit may be further configured to modulate a third frequency of the alternating signal of the third waveform to ensure that the third frequency is related to the beat frequency (controlled by modulating the first and/or second frequency) by an integer number.

The control unit may also or alternatively be configured to modulate an amplitude of the first waveform and/or modulate an amplitude of the second waveform and/or modulate an amplitude of the third waveform. This implies that a location in the nerve having a maximum amplitude of the interferential signal may be controlled. If the amplitude of the first waveform is increased, a location having maximum amplitude of interference between the first waveform and the second waveform is moved closer to the second pair of electrodes, and vice versa. In addition, by modulating the amplitude of the third waveform, a location or area within the nerve or part of the nervous system wherein the interferential signal is sufficiently strong to trigger a signal or block a signal within the nerve or the part of the nervous system may be controlled. Thus, by modulating the amplitude of the first waveform, the amplitude of the second waveform and/or the amplitude of the third waveform, a location within the nerve or the part of the nervous system where stimulation is provided may be controlled. This implies that the control unit may for instance be configured to selectively stimulate a particular fascicle or even a particular axon or group of axons within the nerve.

The control unit may also or alternatively be configured to modulate a time instant for start of the first waveform and/or a time instant for start of the second waveform and/or a time instant for start of the third waveform, respectively. Thus, the control unit may be configured to control a phase relation between the stimulation signals, which further controls a waveform of the interferential signal.

According to an embodiment, the device further comprises at least a fourth pair of electrodes configured to be arranged in a fourth location in relation to the nerve or the part of the nervous system, wherein the stimulation generating unit is configured to generate a fourth waveform comprising a fourth frequency to the fourth pair of electrodes, wherein the fourth frequency is related to the beat frequency by an integer number.

Thus, in addition to the stimulation generating unit generating a first waveform, a second waveform, a third waveform, the stimulation generating unit may be further configured to generate additional waveforms, such as the fourth waveform, to be output to additional pairs of electrodes.

The stimulation generating unit may comprise an additional separate stimulation generating module (and any further stimulation generating modules) that may be individually controlled to provide the fourth waveform independently of the first waveform, the second waveform, and the third waveform.

The fourth waveform may contribute to forming of the interferential stimulation so as to allow a further improved control of the interferential stimulation. The use of additional waveforms, such as the fourth waveform, may thus allow an improved control of the interferential stimulation.

The frequency, amplitude, and/or time instant for start of the fourth waveform may be controlled independently from control of the first waveform, the second waveform, and the third waveform. The interferential stimulation may thus be controlled to obtain an arbitrary waveform of the interferential stimulation and to provide accurate location within the nerve wherein stimulation of the nerve is provided by the interferential stimulation.

Similar to the third frequency, the fourth frequency is related to the beat frequency by an integer number. Thus, the fourth frequency may be the same as the third frequency. Alternatively, the fourth frequency may be different from the third frequency while still being related to the beat frequency by an integer number.

The fourth pair of electrodes may be arranged at a different location in relation to the nerve or the part of the nervous system compared to the first pair of electrodes, the second pair of electrodes, and the third pair of electrodes. All of the electrodes of the first pair of electrodes, the second pair of electrodes, the third pair of electrodes, and the fourth pair of electrodes may be arranged in relation to a common cross-section of the nerve or the part of the nervous system. According to an alternative, the electrodes may also be displaced in relation to each other, e.g., along a longitudinal direction of the nerve.

Output of the fourth waveform to the fourth pair of electrodes may be used such that the device may control interferential stimulation based on the first waveform, the second waveform, the third waveform, and the fourth waveform being simultaneously output. However, the device may additionally or alternatively control interferential stimulation by the control unit selectively activating electrodes so as to select which pairs of electrodes to be used for defining interferential stimulation.

Hence, according to an embodiment, the stimulation generating unit is configured to be controlled to selectively output the third waveform to the third pair of electrodes or the fourth waveform to the fourth pair of electrodes for controlling the interferential stimulation in the nerve or the part of the nervous system.

Thus, the device may select which of the third pair of electrodes or the fourth pair of electrodes that are active. Since the third pair of electrodes and the fourth pair of electrodes are differently placed in relation to the nerve or the part of the nervous system, the selective output of the third waveform or the fourth waveform may control a location in the nerve or the part of the nervous system that is stimulated.

According to an embodiment, the stimulation generating unit is configured to generate a first frequency and a second frequency, respectively, being in a range of 500 Hz - 1 MHz, such as 50 kHz - 1 MHz.

Thus, the stimulation generating unit may generate signals of the first waveform and the second waveform having a relatively high frequency. The frequency of the signal in the first waveform and the frequency of the signal in the second waveform may be so high as not to affect, or minimally affect, the nerve or the part of the nervous system. By selecting these frequencies to be high, such as in the range of 5 kHz - 1 MHz or in the range of 50 kHz - 1 MHz, the nerve is not stimulated by the first waveform and the second waveform individually. Rather, stimulation of the nerve may be provided solely by the interferential signal between the first waveform and the second waveform.

The first frequency and the second frequency may, while not causing generation of signals in the nerve or part of the nervous system, be configured to block a signal transported by the nerve or the part of the nervous system. In this case, the first frequency and the second frequency may for instance be in a range of 5 - 50 kHz.

Also, the amplitude of the signal in the first waveform and the signal in the second waveform may be relatively low compared to the amplitude of the interferential signal, such that the relatively low amplitude signal in the first and second waveforms, respectively, does not affect the nerve. The first frequency and the second frequency may preferably not be too high so that a relatively small difference in the frequencies may still be accurately defined. Thus, the first frequency and the second frequency may be lower than 1 MHz, such as lower than 100 kHz.

According to an embodiment, the stimulation generating unit is configured to generate the first frequency and the second frequency with a difference between the first frequency and the second frequency being in a range of 1 Hz - 10 kHz, such as 1 - 5 kHz.

Thus, the beat frequency may be in the range of 1 Hz - 10 kHz or in the range of 1 - 5 kHz. The use of a beat frequency in these ranges may be suitable for providing stimulation of the nerve or the part of the nervous system. If the beat frequency is very low, it may be difficult to accurately define the interferential signal in relation to high frequencies of the first and the second frequencies. Thus, the beat frequency may preferably be higher than 1 Hz or higher than 1 kHz. If the beat frequency is too high, the interferential signal may not provide a strong effect for stimulation of the nerve or the part of the nervous system. Thus, the beat frequency may preferably be lower than 10 kHz or lower than 5 kHz.

The beat frequency may be selected in relation to a desired stimulation of the nerve or the part of the nervous system, such as in relation to a particular stimulation paradigm to be used.

According to an embodiment, the stimulation generation unit is configured to generate the first waveform, the second waveform, and the third waveform with an amplitude of the third waveform being equal to or larger than an amplitude of the first waveform and an amplitude of the second waveform.

This may imply that the third waveform may efficiently modulate the interference between the first waveform and the second waveform for forming a sufficiently strong interferential signal for stimulating the nerve or the part of the nervous system.

In particular, if the device is configured to use only the first waveform, the second waveform, and the third waveform for defining interferential stimulation, the amplitude of the third waveform may preferably be equal to or larger than an amplitude of the first waveform and an amplitude of the second waveform. If additional waveforms are used, other relations between the amplitudes may be used.

According to an embodiment, the first waveform, the second waveform, and the third waveform are sinusoidal signals.

This implies that first pulses and the second pulses do not deliver net charges to the living being. Hence, accumulation of net charges in the living being is avoided, which may otherwise negatively affect the living being.

According to an embodiment, the device comprises a feedback sensor configured to provide feedback on stimulation of the nerve or the part of the nervous system, wherein the stimulation generating unit is configured to be controlled in dependence of the feedback.

The first waveform, the second waveform, and the third waveform may all be current signals, i.e., constituting a first current waveform, a second current waveform and a third current waveform, respectively. The feedback sensor may in turn be configured for sensing a voltage amplitude at the first pair of electrodes, at the second pair of electrodes, and at the third pair of electrodes, respectively, wherein the stimulation generation unit is configured to be controlled for controlling the voltage amplitude(s) and controlling the interferential stimulation.

The first current waveform, the second current waveform, and the third current waveform generate a corresponding voltage across the first pair of electrodes, across the second pair of electrodes, and across the third pair of electrodes, respectively. This voltage may be sensed using the same pair of electrodes for providing the current waveforms into the nerve or using another pair of electrodes arranged in close vicinity to the first pair of electrodes, the second pair of electrodes, and the third pair of electrodes, respectively.

By monitoring the voltage amplitude at the first pair of electrodes, the second pair of electrodes, and the third pair of electrodes, respectively, feedback on the stimulation may be provided. The stimulation generating unit may be configured to be controlled for controlling the amplitude of the first current waveform, the second current waveform, and the third current waveform, respectively, which in turn controls the corresponding voltage amplitude.

The stimulation generating unit may be configured to be controlled by receiving control signals from the control unit. The control unit may receive the feedback from the feedback sensor and may analyze the feedback in relation to desired / expected feedback measurements and may generate control signals to the stimulation generating unit in accordance with analysis of the feedback.

The interferential stimulation may be controlled so as to control a location within the nerve where stimulation is provided. This implies that the sensor may provide feedback for allowing the control unit to accurately control the location within the nerve where stimulation is provided.

It should be realized that the feedback sensor need not necessarily measure a voltage amplitude at the pairs of electrodes in order to provide feedback on the stimulation of the nerve.

According to an embodiment, the feedback sensor is configured to monitor an effect of stimulation of the nerve or the part of the nervous system by monitoring a specific biomarker.

Thus, the feedback sensor may be configured to monitor effect of stimulation. The stimulation of the nerve or the part of the nervous system may thus be controlled in dependence of whether a desired effect of the stimulation is achieved.

The monitoring of the effect of stimulation may be made in different ways depending on the effect to be achieved, e.g., depending on a type of organ to be affected by stimulation.

According to an embodiment, the feedback sensor is configured to monitor a heart rate and/or a breathing rate as a specific biomarker. According to another embodiment, the feedback sensor is configured to monitor mechanical effects caused by the stimulation, such as muscle activity or intestine mobility as a specific biomarker. According to yet another embodiment, the feedback sensor is configured to monitor an inflammatory biomarker as a specific biomarker.

Thus, the feedback from the feedback sensor may indicate whether the desired effect is achieved, which may provide an indication whether a correct location in the nerve or the part of the nervous system is stimulated. For instance, since the vagus nerve provides signals to many different parts of the body, feedback may be used when providing stimulation of the vagus nerve such that stimulation of the nerve is controlled through interferential stimulation to selectively stimulate a portion of the nerve for providing the desired effect of the stimulation.

According to an embodiment, the stimulation generating unit is configured to generate a first intermittent current waveform, a second intermittent current waveform, and a third intermittent current waveform forming the first waveform, the second waveform and the third waveform, respectively.

Thus, a signal may be intermittently provided to the first pair of electrodes, a signal may be intermittently provided to the second pair of electrodes, and a signal may be intermittently provided to the third pair of electrodes. The first waveform, the second waveform, and the third waveform may each comprise a sequence of pulses and need not be continuous over a plurality of pulses. Rather, the first pair of electrodes, the second pair of electrodes, and the third pair of electrodes may only receive signals at time instances when stimulation of the nerve is to take place.

Thus, the first intermittent current waveform may provide a non-zero signal or high amplitude signal within first pulses of a sequence of first pulses and may provide a zero-level signal between the first pulses within the sequence of first pulses. Also, the second intermittent current waveform may provide a non-zero signal or high amplitude signal within second pulses of a sequence of second pulses and may provide a zero-level signal between the second pulses within the sequence of second pulses. Further, the third intermittent current waveform may provide a non-zero signal or high amplitude signal within third pulses of a sequence of third pulses and may provide a zero-level signal between the third pulses within the sequence of third pulses. It should be realized that instead of a zero-level signal a low amplitude signal may be provided between the first pulses, between the second pulses and between the third pulses, respectively. The low amplitude signal may have such a low amplitude that no stimulation of the nerve takes place through interferential stimulation and the amplitude may also be low so as not to draw a lot of energy.

The first intermittent current waveform, the second intermittent current waveform, and the third intermittent current waveform may thus be called intermittent in that the waveforms intermittently have sufficiently high amplitude to cause interferential stimulation and therebetween have a very low or zero amplitude to reduce power consumption.

Thus, thanks to the stimulation generating unit being configured to provide intermittent current waveforms, the stimulation of the nerve may be provided in an energy-efficient manner. The stimulation generating unit need only output signals during the first pulses, the second pulses, and the third pulses, respectively.

This implies that the device facilitates use of an implanted device (for which energy saving is particularly important in order to ensure long lifetime of the implanted device or avoid frequent charging of the implanted device) with stimulation paradigms that include a nerve being stimulated in very long sessions (such as up to 30 minutes) comprising sequences of pulses being provided in plural periods separated by "off time" when no stimulation is provided.

The first intermittent current waveform may comprise a varying signal defining the first frequency within each of the first pulses. Thus, the first frequency is provided within the first pulse. This should not be confused with a periodicity of the pulses, defining a time interval between two different pulses within the sequence of first pulses.

Similarly, the second intermittent current waveform comprises a varying signal defining the second frequency within each of the second pulses. Thus, the second frequency is provided within the second pulse. This should not be confused with a periodicity of the pulses, defining a time interval between two different pulses within the sequence of second pulses.

Similarly, the third intermittent current waveform comprises a varying signal defining the third frequency within each of the third pulses. Thus, the third frequency is provided within the third pulse. This should not be confused with a periodicity of the pulses, defining a time interval between two different pulses within the sequence of third pulses.

According to a third aspect, there is provided a method for controlling stimulation of a nerve or a part of a nervous system of a living being, said method comprising: providing a first control signal for controlling generation of a first waveform comprising a first frequency to a first pair of electrodes arranged in a first location in relation to the nerve or the part of the nervous system; providing a second control signal for controlling generation of a second waveform comprising a second frequency to a second pair of electrodes arranged in a second location in relation to the nerve or the part of the nervous system; providing a third control signal for controlling generation of a third waveform comprising a third frequency to a third pair of electrodes arranged in a third location in relation to the nerve or the part of the nervous system; wherein the control signals control generation of the first waveform and the second waveform with a difference between the first frequency and the second frequency defining a beat frequency and control generation of the third frequency being related to the beat frequency by an integer number.

Effects and features of this second aspect are largely analogous to those described above in connection with the first aspect. Embodiments mentioned in relation to the second aspect are largely compatible with the first aspect.

The method relates to controlling generation of signals that may be used for stimulation of the nerve or the part of the nervous system. The method provides control signals for controlling timing of generation of the first waveform, the second waveform, and the third waveform, and controlling frequencies of the waveforms. Thus, the method provides control of a device for generating waveforms. The method does not relate to actual output of the waveforms and is therefore not related to a method of treatment of a living being.

Thanks to the providing control signals for controlling generation of the first waveform, the second waveform, and the third waveform with the third waveform comprising the third frequency being related to the beat frequency by an integer number, the method allows control of generation of stimulation signals such that the third waveform may constructively interfere with the first and second waveforms such that a stimulation signal in a particular portion of the nerve or the part of the nervous system may be amplified and cause interferential stimulation. The third waveform thus allows accurate selection of a location in the nerve or the part of the nervous system in which interferential stimulation will occur.

According to an embodiment, the method further comprises modulating the first waveform, the second waveform, and the third waveform by modulating the first frequency, the second frequency, and the third frequency, respectively, by modulating an amplitude of the first waveform, the second waveform, and the third waveform, respectively, and/or by modulating a time instant for start of the first waveform, the second waveform, and the third waveform, respectively.

This implies that the control of stimulation may accurately control generation of the first waveform, the second waveform, and the third waveform for accurately defining interferential stimulation.

### Brief description of the drawings

The above, as well as additional objects, features and advantages of the present inventive concept, will be better understood through the following illustrative and non-limiting detailed description, with reference to the appended drawings. In the drawings like reference numerals will be used for like elements unless stated otherwise.
Fig. 1 is a schematic view illustrating interferential stimulation of a nerve.
Fig. 2 is a schematic view of a device according to an embodiment.
Figs 3a-3g are schematic views illustrating interferential signals in the nerve based on different arrangement of electrodes of the device and different waveforms output to the electrodes.
Fig. 4 is a schematic view of a system incorporating the device.
Fig. 5 is a flow chart of a method according to an embodiment.

### Detailed description

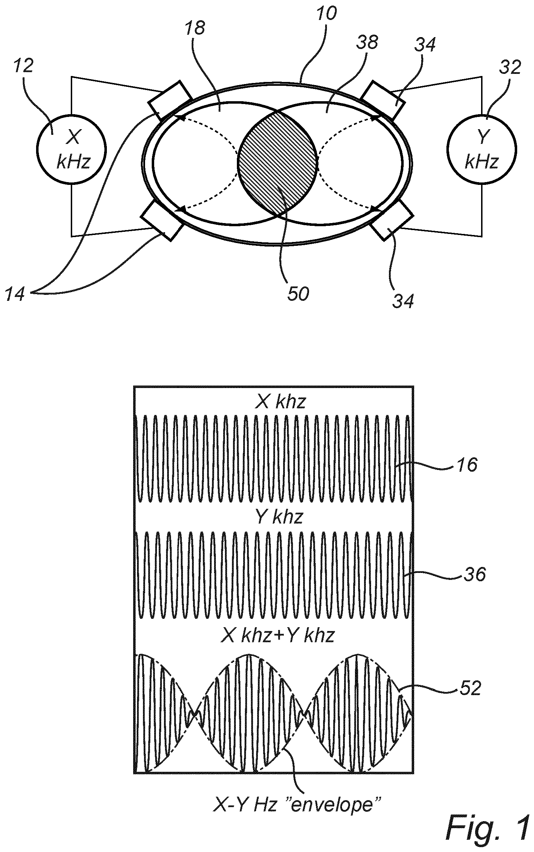

Referring now to Fig. 1, interferential stimulation of a nerve 10 will be generally described.

As illustrated in Fig. 1, a first current source 12 is associated with a first pair of electrodes 14 and a second current source 32 is associated with a second pair of electrodes 34. The first pair of electrodes 14 is mounted in a first relation to an outer surface of the nerve 10 and the second pair of electrodes 34 is mounted in a second relation to the outer surface of the nerve 10.

The first current source 12 generates a first signal 16 with a first frequency X kHz and the second current source 32 generates a second signal 36 with a second frequency Y kHz. As illustrated in Fig. 1, the first signal provided to the first pair of electrodes 14 causes an electric field to be generated in a first portion 18 of the nerve 10 and the second signal 36 provided to the second pair of electrodes 34 causes an electric field to be generated in a second portion 38 of the nerve 1. The electric fields overlap in an overlapping portion 50, wherein the first signal 16 interferes with the second signal 36 to generate an interferential signal 52.

The interferential signal 52 has a frequency content including the first frequency and the second frequency. Further, the amplitude of the interferential signal 52 is modulated by a difference between the first frequency and the second frequency, X-Y, defining a beat frequency of the interferential signal 52.

The beat frequency may be much lower than the first and second frequencies. Further, the amplitude of the interferential signal 52 may be larger than individual amplitudes of the first signal 16 and the second signal 36, respectively, thanks to constructive interference of the signals.

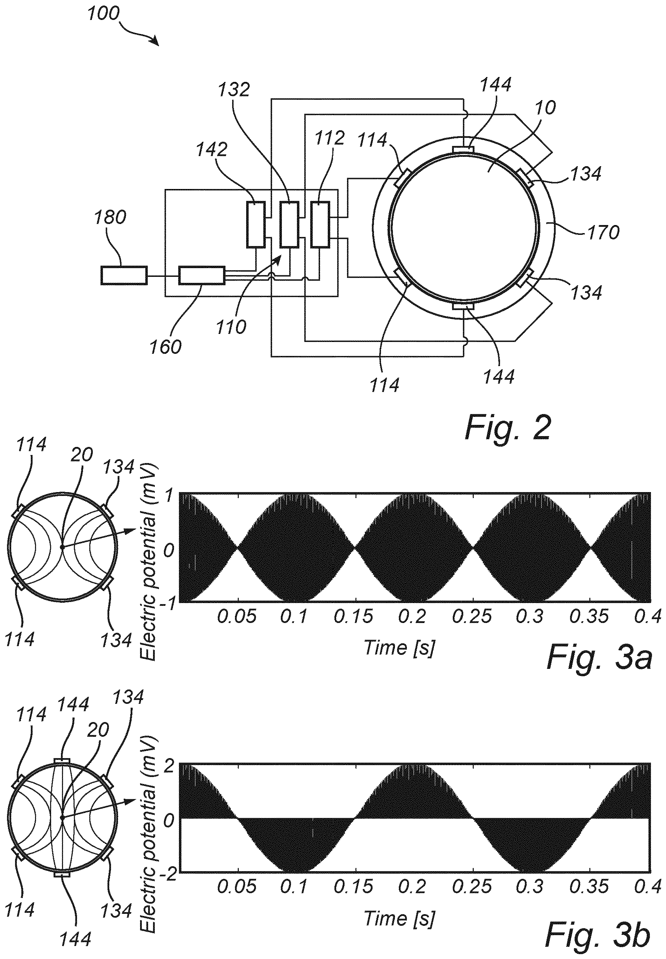

Referring now to Fig. 2, a device 100 for stimulating a nerve 10 according to an embodiment is illustrated. Although interferential stimulation of a nerve 10 will be described hereinbelow, it should be realized that the interferential stimulation may alternatively be applied to another part of the nervous system, such as a part of the peripheral nervous system, e.g., to a ganglion. For simplicity and brevity, only stimulation of a nerve 10 will be described below.

The device 100 is configured to generate a third signal for modulating interference between a first signal and a second signal, as described above, such that the interferential signal within the nerve 10 may be accurately controlled for accurate selection of a location within the nerve 10 in which interferential stimulation is caused.

The device 100 comprises a stimulation generating unit 110, which may comprise separate stimulation generating modules, each dedicated to generating stimulation signals to a respective pair of electrodes. Alternatively, the stimulation generating unit 110 may be a single unit configured to generate and output the stimulation signals to the electrodes.

In Fig. 2, the device 100 is shown with the stimulation generating unit 110 comprising a first stimulation generating module 112, a second stimulation generating module 132 and a third stimulation generating module 142. The first stimulation generating module 112, the second stimulation generating module 132 and the third stimulation generating module 142 are separate from each other which may facilitate that the generation and output of stimulation signals from the stimulation generating modules 112, 132, 142 may be individually provided without crosstalk between the stimulation generating modules 112, 132, 142.

Each stimulation generating module may comprise a current generator for generating a current signal. The current generator may comprise an oscillator, which is configured to generate an alternating signal and an amplifier, which is configured to receive the alternating signal from the oscillator and output an amplified signal. The stimulation generating module may be controlled, e.g., for controlling a frequency of the alternating signal output by the oscillator or for controlling a gain of the amplifier. As realized by the person skilled in the art, the stimulation generating module may be implemented in numerous other ways for generating an alternating signal.

The first stimulation generating module 112 may comprise a first current generator, which may be connected to a first pair of electrodes 114. The first current generator may be configured to generate a first waveform and to output the first waveform to the first pair of electrodes 114. The first pair of electrodes 114 are arranged in a first location in relation to the nerve 10.

The second stimulation generating module 132 may comprise a second current generator, which may be connected to a second pair of electrodes 134. The second current generator may be configured to generate a second waveform and to output the second waveform to the second pair of electrodes 134. The second pair of electrodes 134 are arranged in a second location in relation to the nerve 10.

The third stimulation generating module 142 may comprise a third current generator, which may be connected to a third pair of electrodes 144. The third current generator may be configured to generate a third waveform and to output the third waveform to the third pair of electrodes 144. The third pair of electrodes 144 are arranged in a third location in relation to the nerve 10.

The first stimulation generating module 112 and the second stimulation generating module 132 are configured to generate and output the first waveform comprising a first frequency and the second waveform comprising a second frequency with a difference in the first and second frequencies such that the difference between the first frequency and the second frequency defines a beat frequency in interference within the nerve 10 of the first waveform and the second waveform.

The third stimulation generating module 142 is further configured to generate and output the third waveform comprising a third frequency that is related to the beat frequency by an integer number.

The third waveform modulates the beat frequency signal defined by interference of the first waveform and the second waveform such that an amplitude of the beat frequency signal is substantially increased. In particular, thanks to the third frequency being related to the beat frequency by an integer number, the third waveform may be matched to the beat frequency signal in order to amplify the beat frequency signal and to control stimulation of the nerve 10. A phase of the third waveform may be controlled in relation to a phase of the beat frequency signal such that constructive interference may be achieved in a desired location so as to cause interferential stimulation in the desired location of the nerve 10.

The first pair of electrodes 114, the second pair of electrodes 134, and the third pair of electrodes 144 may be configured to be arranged in relation to a cross-section of the nerve 10 perpendicular to a longitudinal direction of the nerve 10. The first pair of electrodes 114 may be configured to be arranged at an opposite side of a circumference of the nerve 10 to the second pair of electrodes 134. However, individual electrodes within a pair of electrodes 114, 134 may still be configured to be arranged with a distance in-between such that the current waveform received by the electrodes will propagate through nerve tissue between the electrodes. The electrodes of the third pair of electrodes 144 may be configured to be arranged at opposite sides of the circumference of the nerve 10 with each of the electrodes of the third pair of electrodes 144 being arranged between one of the electrodes of the first pair of electrodes 114 and one of the electrodes of the second pair of electrodes 134 along the circumference of the nerve 10.

Thus, the first pair of electrodes 114 may be associated with a first part of the circumference of the nerve 10 and the second pair of electrodes 134 may be associated with a second part of the circumference of the nerve 10. This implies that the portions of the cross-section of the nerve in which the first waveform and the second waveform, respectively, define electric fields are quite different so as to allow interferential stimulation deep within the nerve 10.

However, according to an alternative, the electrodes of the first pair of electrodes 114 may be configured to be alternatingly arranged with the electrodes of the second pair of electrodes 134 and the electrodes of the third pair of electrodes 144 around the circumference of the nerve 10. Different arrangements of the first pair of electrodes 114, the second pair of electrodes 134 and the third pair of electrodes 114 in relation to the nerve 10 may be used depending on a location within the nerve 10 at which stimulation is desired.

According to another alternative, the electrodes of the first pair of electrodes 114 may be displaced in relation to each other along a longitudinal direction of the nerve 10. The electrodes of the second pair of electrodes 134 may also be displaced in relation to each other along a longitudinal direction of the nerve 10. Also, the electrodes of the third pair of electrodes 144 may be displaced in relation to each other along a longitudinal direction of the nerve 10. The first pair of electrodes 114 may be displaced from the second pair of electrodes 134 and the third pair of electrodes 144 along the circumference of the nerve 10, but the electrodes of the first, second, and third pairs of electrodes 114, 134, 144 may be arranged at common positions in relation to the longitudinal direction of the nerve 10. This implies that an interferential signal 152 may be provided along the longitudinal direction of the nerve 10, which may be useful for stimulating the nerve 10. However, it may be more difficult to select a desired location within the cross-section of the nerve 10 when the electrodes of the pairs of electrodes 114, 134, 144 are displaced in the longitudinal direction of the nerve 10.

As illustrated in Fig. 2, the device 100 may comprise a carrier 170, such as in the form of a cuff, in which the first pair of electrodes 114, the second pair of electrodes 134, and the third pair of electrodes 144 are mounted. The cuff 170 may facilitate arrangement of the first, second, and third pairs of electrodes 114, 134, 144 in contact with a surface of the nerve 10.

The first pair of electrodes 114, the second pair of electrodes 134, and the third pair of electrodes 144 may be mounted in the cuff 170 with a fixed relation between the electrodes. This implies that the cuff 170 may provide a well-defined relation between the electrodes providing a simple manner of arranging all the electrodes with a desired relationship to each other and to the nerve 10.

The device 100 may further comprise a control unit 160. The control unit 160 may be configured to provide control signals to the stimulation generating unit by providing control signals to the first stimulation generating module 112, the second stimulation generating module 132, and the third stimulation generating module 142 for controlling generation of the first waveform, generation of the second waveform, and generation of the third waveform, respectively. The control unit 160 may further be configured to synchronize the first stimulation generating module 112, the second stimulation generating module 132, and the third stimulation generating module 142 such that the first waveform, the second waveform and the third waveform are output simultaneously or almost simultaneously with a desired time relation to each other. The control unit 160 may thus ensure that a desired interferential signal is generated for providing desired interferential stimulation of the nerve 10.

The control unit 160 may be configured to provide control signals to the first current generator, to the second current generator, and to the third current generator, respectively, for controlling the generation of the first waveform, the generation of the second waveform, and the generation of the third waveform, respectively. The control signals to the first current generator, to the second current generator, and to the third current generator may modulate the first waveform, the second waveform, and the third waveform, respectively, for controlling the interferential stimulation provided by the device 100.

The control unit 160 may be configured to provide control signals for modulating the first frequency of the first waveform and/or the second frequency of the second waveform. This implies that the beat frequency may be changed. Thus, the control unit 160 may further be configured to provide control signals for modulating the third frequency of the third waveform such that the third frequency relates to the beat frequency by an integer number.

The control unit 160 may be configured to provide control signals for modulating an amplitude of the first waveform and/or an amplitude of the second waveform and/or an amplitude of the third waveform. This implies that a location within the nerve 10 having a maximum amplitude of the interferential signal may be moved. The location having maximum amplitude of the interferential signal is located closer to a pair of electrodes outputting a weaker signal than to a pair of electrodes outputting a stronger signal.

The control unit 160 may be configured to provide control signals for modulating a time instant for start of the first waveform and/or a time instant for start of the second waveform and/or a time instant for start of the third waveform. Thus, a phase relationship between the first waveform, the second waveform, and the third waveform may be controlled for controlling a waveform of the interferential signal.

The control unit 160 may comprise a stimulation controller which is configured to generate and transmit the control signals to the first stimulation generating module 112, the second stimulation generating module 132, and the third stimulation generating module 142.

According to an embodiment, the stimulation controller may comprise a processing unit for executing an application program for controlling the generation and timing of control signals. The stimulation controller may further comprise a memory storing the application program and storing settings for controlling the generation and timing of control signals. The stimulation controller may be configured to receive new settings for altering the control by the stimulation controller. The stimulation controller may also or alternatively comprise several versions of settings to allow selection of different settings depending on a desired stimulation. The stimulation controller may further comprise a clock or may be configured to receive a clock signal as input for timing of control signals.

According to an alternative, the stimulation controller may comprise an application-specific integrated circuit (ASIC) for controlling the generation and timing of control signals.

The first stimulation generating module 112 and the second stimulation generating module 132 may be configured to generate the first waveform and the second waveform, respectively, having a first frequency and a second frequency, respectively, in a range of 500 Hz - 1 MHz, such as 50 kHz - 1 MHz.

The first stimulation generating module 112 and the second stimulation generating module 132 may further be configured to generate the first waveform and the second waveform with a difference in frequency in a range of 1 Hz - 10 kHz, such as 1 - 5 kHz. Thus, the beat frequency of the interferential signal may be in the range of 1 Hz - 10 kHz, such as 1 - 5 kHz.

The first frequency and the second frequency generated by the first stimulation generating module 112 and the second stimulation generating module 132 may thus be sufficiently high so as not to affect the nerve 10, such as being higher than 500 Hz or higher than 50 kHz. The difference between the first frequency and the second frequency, i.e., the beat frequency, may be set so as to be adapted to provide proper stimulation of the nerve 10.

The third stimulation generating module 142 may be configured to generate the third waveform comprising a third frequency which is related to the beat frequency by an integer number.

According to an embodiment, the beat frequency may be two times larger than the third frequency. The beat frequency being two times larger than the third frequency may be particularly advantageous in forming a desired interferential signal in the nerve 10.

However, it should be realized that the third frequency may be related to the beat frequency by an integer number in other ways. For instance, the third frequency may be equal to the beat frequency, or the third frequency may be two, three or four times larger than the beat frequency. This may still allow the third waveform to modulate the interference between the first and second waveforms so as to form an interferential signal that may efficiently cause stimulation in the nerve 10.

Thus, according to embodiments, the third frequency of the third waveform may be in the range of 0.5 Hz - 20 kHz, such as 500 Hz - 10 kHz.

The signal the first waveform, the signal of the second waveform, and the signal of the third waveform may be sinusoidal signals. This implies that no net charges are delivered to the living being by the first waveform, the second waveform, and the third waveform, respectively. Hence, accumulation of net charges in the living being is avoided, which may otherwise negatively affect the living being.

The stimulation controller may be configured to control the first stimulation generating module 112, the second stimulation generating module 132, and the third stimulation generating module 142 to control the device 100 to provide stimulation of the nerve 10 according to a stimulation paradigm. The stimulation controller may thus store settings of the stimulation paradigm and may be configured to control the first stimulation generating module 112, the second stimulation generating module 132, and the third stimulation generation module 142 to generate and output the first waveform, the second waveform, and the third waveform for providing the desired stimulation paradigm to the nerve 10.

The device 100 may comprise a feedback sensor 180. The feedback sensor 180 is configured to measure a property related to the stimulation of the nerve 10. The feedback sensor 180 may provide measurement data to the control unit 160, which may use the measurement data in order to control the stimulation generating unit 110 in dependence of the measurement data.

The feedback sensor 180 may be configured to sense a voltage amplitude at the first pair of electrodes 114, at the second pair of electrodes 134, and at the third pair of electrodes 144, respectively, using the same electrodes used for providing the first, second, and third waveforms or using additional electrodes in vicinity of first pair of electrodes 114, the second pair of electrodes 134, and the third pair of electrodes 144, respectively.

The control unit 160 may analyze the measurement data, such as the sensed voltage amplitudes. The control unit 160 may store desired values of the voltage amplitudes to be used in the stimulation of the nerve 10 such that the control unit 160 may control the stimulation generating modules 112, 132, 142 in dependence of the sensed voltage amplitudes such that the desired values are reached. The stored desired values may represent calibration values corresponding to the device 100 providing stimulation of a desired location in the nerve 10.

The control unit 160 may alternatively determine a location in the nerve 10 being stimulated based on the sensed voltage amplitudes in order to control the stimulation generating modules 112, 132, 142 such that a desired location is stimulated.

The feedback sensor 180 may alternatively be configured to measure a property related to an effect of the stimulation of the nerve 10. Thus, the feedback sensor 180 may be arranged to measure a property in a separate location from the location where stimulation of the nerve 10 takes place. The feedback sensor 180 may be thus be arranged in a separate housing, which may for instance be implanted in the body of the living being or may be worn on the body of the living being depending on the property to be measured. The feedback sensor 180 may then communicate with the control unit 160 through wired or wireless communication.

The feedback sensor 180 may be configured to monitor a specific biomarker in order to monitor an effect of the stimulation of the nerve 10.

According to an embodiment, the feedback sensor 180 is configured to monitor a heart rate and/or a breathing rate as a specific biomarker. According to another embodiment, the feedback sensor 180 is configured to monitor mechanical effects caused by the stimulation, such as muscle activity or intestine mobility as a specific biomarker. According to yet another embodiment, the feedback sensor 180 is configured to monitor an inflammatory biomarker as a specific biomarker.

The control unit 160 may be configured to analyze the feedback from the feedback sensor 180 in order to determine whether the desired effect of the stimulation of the nerve 10 is achieved. Such analysis may provide an indication whether a correct location in the nerve 10 is stimulated.

For instance, the vagus nerve provides signals to many different parts of the body. The analysis of the feedback from the feedback sensor 180 may reveal whether stimulation by the device 100 provides stimulation of a correct location in the vagus nerve such that the desired part of the body is affected. Hence, the feedback may be used for monitoring that the correct location in the vagus nerve is stimulated, such that the device 100 is controlled to selectively stimulate a portion of the vagus nerve for providing the desired effect of the stimulation.

Referring now to Figs 3a-3g, interferential signals achieved in the nerve 10 are discussed. It should be realized that the interferential signals are based on simulations and may not correspond exactly to interferential signals in an actual nerve 10. However, the interferential signals in Figs 3a-3g illustrate how stimulation in the nerve 10 may be controlled.

In Fig. 3a, a first waveform is provided into the nerve 10 by the first pair of electrodes 114 and a second waveform is provided by the second pair of electrodes 134, wherein the first pair of electrodes 114 and the second pair of electrodes 134 are arranged at different locations in relation to a circumference of the nerve 10 as illustrated in Fig. 3a.

The first waveform is a sinusoidal signal having a first frequency of 1 kHz and an amplitude of 1 mV. The second waveform is a sinusoidal signal having a second frequency of 1.01 kHz and an amplitude of 1 mV. The first and second waveforms are synchronized with a common phase relation.

In Fig. 3a, the interferential signal between the first waveform and the second waveform at a location 20 in the nerve 10 centrally between the first pair of electrodes 114 and the second pair of electrodes 134 is illustrated. Attenuation of each of the first waveform and the second waveform between the location 20 and the first pair of electrodes 114 and the second pair of electrodes 134, respectively, is 0.5. As shown in Fig. 3a, the interferential signal exhibits a beat frequency of 10 Hz varying between a maximum interference and a minimum interference wherein the first and second waveforms cancel each other.

In Fig. 3b, in addition to the first and second waveforms illustrated in Fig. 3a, a third waveform is provided into the nerve 10 by the third pair of electrodes 144. The third pair of electrodes 144 are arranged at a different location in relation to a circumference of the nerve 10 compared to the first pair of electrodes 114 and the second pair of electrodes 134. The electrodes of the third pair of electrodes 144 are arranged on opposite sides of the nerve 10 and each of the electrodes 144 are arranged between an electrode of the first pair of electrodes 114 and an electrode of the second pair of electrodes 134 along the circumference of the nerve 10 as illustrated in Fig. 3b.

The third waveform is a sinusoidal signal having a third frequency of 5 Hz and an amplitude of 2 mV. Thus, the beat frequency is two times larger than the third frequency. The third waveform is provided with a 90° phase offset in relation to the first and second waveforms.

In Fig. 3b, the interferential signal between the first waveform, the second waveform, and the third waveform at the location 20 in the nerve 10 centrally between the first pair of electrodes 114 and the second pair of electrodes 134 is illustrated. Attenuation of the third waveform between the location 20 and the third pair of electrodes 144 is 0.5.

As shown in Fig. 3b, the third waveform modulates the interference between the first waveform and the second waveform such that an amplitude of the interferential signal is substantially increased. In addition, the interferential signal is formed with a similar signal shape as a typical monophasic or biphasic current stimulation which is realized in existing implants.

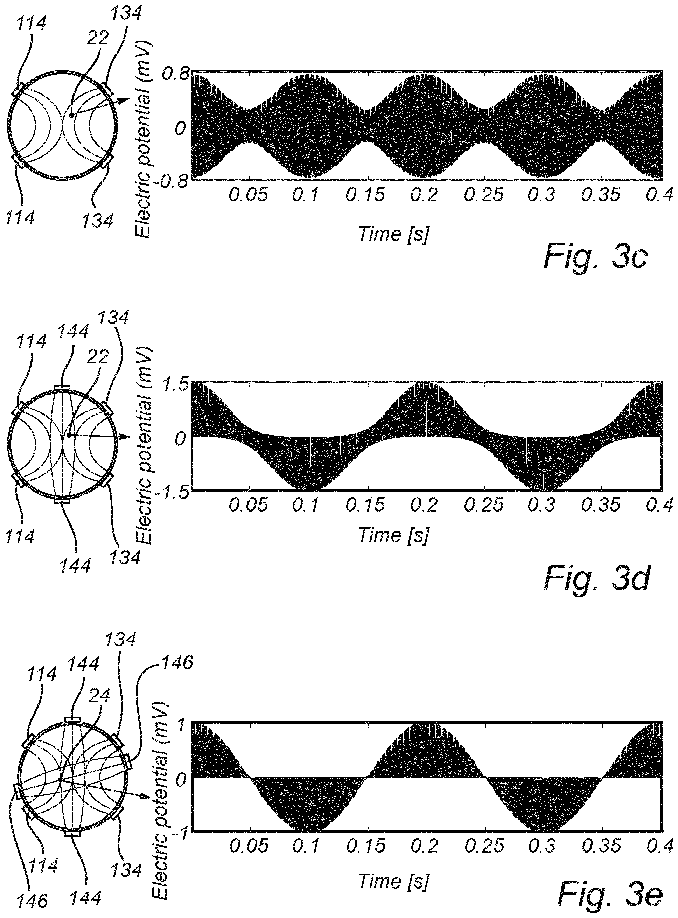

Figs 3c and 3d illustrate similar stimulation effects as illustrated in Figs 3a and 3b. However, in Figs 3c and 3d, stimulation in a different location of the nerve 10 is illustrated using different stimulation parameter settings.

In Fig. 3c, a first waveform is provided into the nerve 10 by the first pair of electrodes 114 and a second waveform is provided by the second pair of electrodes 134, wherein the first pair of electrodes 114 and the second pair of electrodes 134 are arranged at different locations in relation to a circumference of the nerve 10 as illustrated in Fig. 3c, the locations of the first and second pair of electrodes 114, 134 being the same as illustrated in Fig. 3a.

The first waveform is a sinusoidal signal having a first frequency of 1 kHz and an amplitude of 1 mV. The second waveform is a sinusoidal signal having a second frequency of 1.01 kHz and an amplitude of 1 mV. The first and second waveforms are synchronized with a common phase relation.

In Fig. 3c, the interferential signal between the first waveform and the second waveform at a location 22 in the nerve 10 arranged closer to the first pair of electrodes 114 than the second pair of electrodes 134 is illustrated. Attenuation of the first waveform between the location 22 and the first pair of electrodes 114 is 0.5, whereas attenuation of the second waveform between the location 22 and the second pair of electrodes 134 is 0.25. As shown in Fig. 3c, the interferential signal exhibits a beat frequency of 10 Hz varying between a maximum interference and a minimum interference wherein the first and second waveforms do not fully cancel each other out at minimum interference.

In Fig. 3d, in addition to the first and second waveforms illustrated in Fig. 3c, a third waveform is provided into the nerve 10 by the third pair of electrodes 144. The third pair of electrodes 144 are arranged at a different location in relation to a circumference of the nerve 10 compared to the first pair of electrodes 114 and the second pair of electrodes 134, in the same manner as illustrated in Fig. 3b.

The third waveform is a sinusoidal signal having a third frequency of 5 Hz and an amplitude of 1.5 mV. Thus, the beat frequency is two times larger than the third frequency. The third waveform is provided with a 90° phase offset in relation to the first and second waveforms.

In Fig. 3d, the interferential signal between the first waveform, the second waveform, and the third waveform at the location 22 in the nerve 10 closer to the first pair of electrodes 114 than the second pair of electrodes 134 is illustrated. Attenuation of the third waveform between the location 22 and the third pair of electrodes 144 is 0.5.

As shown in Fig. 3d, the third waveform modulates the interference between the first waveform and the second waveform such that an amplitude of the interferential signal is substantially increased. Thus, as illustrated in Figs 3b and 3d, a location in the nerve 10 being stimulated may be controlled such that the stimulation may be steered to different locations in the nerve 10. It should be noted that the stimulation illustrated in Fig. 3d has a lower amplitude than the stimulation illustrated in Fig. 3b and also a gradient of the interferential signal in-between positive and negative halves of a cycle is smaller for the interferential signal illustrated in Fig. 3d than the interferential signal in Fig. 3b. This implies that there is a lower probability of activating nerve fibers at location 22 using the stimulation indicated in Fig. 3d compared to location 20 using the stimulation indicated in Fig. 3b.

As illustrated by the examples in Figs 3b and 3d, the stimulation generation unit 110 may be configured to generate the first waveform, the second waveform, and the third waveform with an amplitude of the third waveform being equal to or larger than an amplitude of the first waveform and an amplitude of the second waveform. This may imply that the third waveform may efficiently modulate the interference between the first waveform and the second waveform for forming a sufficiently strong interferential signal for stimulating the nerve 10.

In Fig. 3e, in addition to the first and second waveforms illustrated in Fig. 3a, a third waveform is provided into the nerve 10 by the third pair of electrodes 144 and a fourth waveform is provided into the nerve 10 by a fourth pair of electrodes 146. The third pair of electrodes 144 are arranged at a different location in relation to the circumference of the nerve 10 compared to the first pair of electrodes 114 and the second pair of electrodes 134, in the same manner as illustrated in Fig. 3b. The fourth pair of electrodes 146 are arranged at yet a different location in relation to the circumference of the nerve 10 compared to the first pair of electrodes 114, the second pair of electrodes 134, and the third pair of electrodes 144. The electrodes of the fourth pair of electrodes 146 are arranged on opposite sides of the nerve 10 and one of the electrodes of the fourth pair of electrodes 146 is arranged between the electrodes of the first pair of electrodes 114 and the other of the electrodes of the fourth pair of electrodes 146 is arranged between the electrodes of the second pair of electrodes 134 along the circumference of the nerve 10 as illustrated in Fig. 3e.

Thus, as illustrated in Fig. 3e, the device 100 may comprise a fourth pair of electrodes 146. The stimulation generating unit 110 may be configured to generate a fourth waveform comprising a fourth frequency to the fourth pair of electrodes 146. The stimulation generating unit 110 may comprise an additional stimulation generating module for separately providing the fourth waveform to the fourth pair of electrodes 146.

As illustrated in Fig. 3e, additional electrodes may thus be used for further controlling the location within the nerve 10 being stimulated. This further facilitates maximizing the interferential signal in a location in the nerve 10 which does not correspond to a location in which interference between the first waveform and the second waveform is strongest.

In Fig. 3e, an interferential signal is illustrated based on interference between the first waveform, the second waveform, the third waveform, and the fourth waveform. Here, the first waveform is a sinusoidal signal having a first frequency of 1 kHz and an amplitude of 1 mV. The second waveform is a sinusoidal signal having a second frequency of 1.01 kHz and an amplitude of 1 mV. The first and second waveforms are synchronized with a common phase relation.

The third waveform in Fig. 3e is a sinusoidal signal having a third frequency of 1 kHz and an amplitude of 0.5 mV. The third waveform is provided with a 180° phase offset in relation to the first and second waveforms. Thus, in this case, the third frequency of the third waveform is equal to the first frequency of the first waveform.

The fourth waveform is a sinusoidal signal having a fourth frequency of 5 Hz and an amplitude of 1 mV. Thus, the beat frequency is two times larger than the fourth frequency. The fourth waveform is provided with a 90° phase offset in relation to the first and second waveforms.

In Fig. 3e, the interferential signal between the first waveform, the second waveform, the third waveform, and the fourth waveform at a location 24 in the nerve 10 arranged closer to the first pair of electrodes 114 along a direction of a line between the electrodes of the fourth pair of electrodes 146 is illustrated. Attenuation of the first waveform between the location 24 and the first pair of electrodes 114 is 0.5, attenuation of the second waveform between the location 24 and the second pair of electrodes 134 is 0.25, attenuation of the third waveform between the location 24 and the third pair of electrodes 144 is 0.5 and attenuation of the fourth waveform between the location 24 and the fourth pair of electrodes 146 is 0.5.

As shown in Fig. 3e, the fourth waveform modulates interference between the first waveform, the second waveform, and the third waveform such that an amplitude of the interferential signal is substantially increased. In addition, the interferential signal is formed with a similar signal shape as a typical monophasic or biphasic current stimulation which is realized in existing implants.

In Fig. 3e, the third waveform has a frequency equal to the first frequency. It should however be realized that both the third waveform and the fourth waveform may be provided to be related to the beat frequency by an integer number.

Also, the stimulation generating unit 110 may be configured to be controlled to selectively output the third waveform to the third pair of electrodes 11 or the fourth waveform to the fourth pair of electrodes 146 for controlling the interferential stimulation in the nerve 10. Thus, a location in the nerve 10 being stimulated may be controlled by selection of which electrodes to be used for outputting waveforms for forming the interferential signal in the nerve 10.

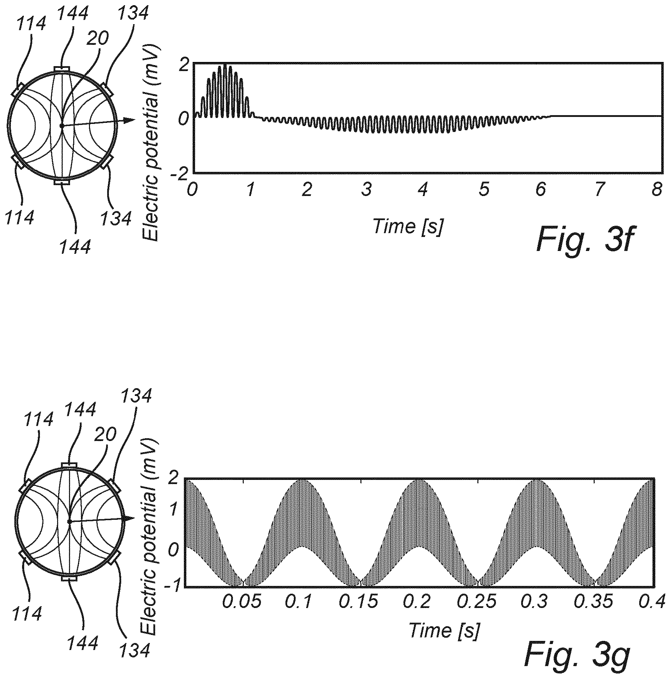

In Fig. 3f, an interferential signal formed by the first waveform, the second waveform, and the third waveform is illustrated, wherein the first pair of electrodes 114, the second pair of electrodes 134 and the third pair of electrodes 144 arranged in the same manner as illustrated in Fig. 3b is used and wherein the interferential signal at the location 20 centrally between the first pair of electrodes 114 and the second pair of electrodes 134 is again illustrated.

In Fig. 3f, the first waveform, the second waveform and the third waveform have different characteristics in a first time interval and a second time interval.

In the first time interval, the first waveform is a sinusoidal signal having a first frequency of 10 kHz and an amplitude of 1 mV. The second waveform is a sinusoidal signal having a second frequency of 11 kHz and an amplitude of 1 mV. The first and second waveforms are synchronized with a common phase relation of 90° at start of the first time interval. The first and the second waveform thus define a beat frequency of 1 kHz. The third waveform is a sinusoidal signal having a third frequency of 500 Hz and an amplitude of 2 mV. Thus, the beat frequency is two times larger than the third frequency. The third waveform is provided with a common phase relation to the first and second waveforms. The first time interval has a duration corresponding to a single period of the beat frequency.

In the second time interval, the first waveform is a sinusoidal signal having a first frequency of 10 kHz and an amplitude of 1 mV. The second waveform is a sinusoidal signal having a second frequency of 10.2 kHz and an amplitude of 1 mV. The first and second waveforms are synchronized with a common phase relation of 90° at start of the first time interval. The first and the second waveform thus define a beat frequency of 200 Hz. The third waveform is a sinusoidal signal having a third frequency of 100 Hz and an amplitude of 2 mV. Thus, the beat frequency is two times larger than the third frequency. The third waveform is provided with a common phase relation to the first and second waveforms. The second time interval has a duration corresponding to a single period of the beat frequency during the second time interval.

In Fig. 3f, the interferential signal between the first waveform, the second waveform, and the third waveform at the location 20 in the nerve 10 centrally between the first pair of electrodes 114 and the second pair of electrodes 134 is illustrated. Attenuation of each of the first waveform, the second waveform and the third waveform between the location 20 and the first pair of electrodes 114, the location 20 and the second pair of electrodes 134 and the location 20 and the third pair of electrodes 144, respectively, is 0.5.

As illustrated in Fig. 3f, the interferential signal may be controlled to mimic traditional charge balanced stimulation. This enables steering stimulation predominantly to a particular location in the nerve 10. The phase of the first waveform, second waveform and third waveform ensure that the interferential signal is positive in the first time interval and negative in the second time interval.

In Fig. 3g, an interferential signal formed by the first waveform, the second waveform, and the third waveform is illustrated, wherein the first pair of electrodes 114, the second pair of electrodes 134 and the third pair of electrodes 144 arranged in the same manner as illustrated in Fig. 3b is used and wherein the interferential signal at the location 20 centrally between the first pair of electrodes 114 and the second pair of electrodes 134 is again illustrated.

The first waveform is a sinusoidal signal having a first frequency of 1 kHz and an amplitude of 1 mV. The second waveform is a sinusoidal signal having a second frequency of 1.01 kHz and an amplitude of 1 mV. The first and second waveforms are synchronized with a common phase relation.

The third waveform is a sinusoidal signal having a third frequency of 10 Hz and an amplitude of 2 mV. Thus, the beat frequency is now equal to the third frequency. The third waveform is provided with a 90° phase offset in relation to the first and second waveforms.

In Fig. 3g, the interferential signal between the first waveform, the second waveform, and the third waveform at the location 20 in the nerve 10 centrally between the first pair of electrodes 114 and the second pair of electrodes 134 is illustrated. Attenuation of each of the first waveform, the second waveform and the third waveform between the location 20 and the first pair of electrodes 114, the location 20 and the second pair of electrodes 134 and the location 20 and the third pair of electrodes 144, respectively, is 0.5.

As shown in Fig. 3g, the third waveform modulates the interference between the first waveform and the second waveform such that an amplitude of the interferential signal is substantially increased. The interferential signal has a different signal shape than illustrated in Fig. 3b. However, the interferential signal in Fig. 3g illustrates that a different relation between the third frequency and the beat frequency may be used while still allowing control of stimulation of the nerve. It should also be realized that other relations between the third frequency and the beat frequency could be used, such as the third frequency being two or three times larger than the beat frequency.

Although the interferential signals in Figs. 3a-3g have been illustrated based on continuous signals of the first waveform, the second waveform and the third waveform, it should be realized that the first waveform, the second waveform and the third waveform may be intermittent signals. Thus, each of the waveforms may comprise a sequence of pulses having a varying signal within each of the pulses with the signal varying at the frequency of the waveform. Thus, stimulation may be provided to the nerve 10 at a time instance when pulses of the waveforms are output, whereas no signal is output by the electrodes between the pulses.

Thanks to the stimulation generating unit 110 being configured to provide intermittent current waveforms, the stimulation of the nerve 10 may be provided in an energy-efficient manner only outputting signals when stimulation is actually to take place. This implies that the device 100 is suitable for being implanted (for which energy saving is particularly important in order to ensure long lifetime of the implanted device or avoid frequent charging of the implanted device). The device 100 may further be used with stimulation paradigms that include a nerve 10 being stimulated in very long sessions (such as up to 30 minutes) comprising sequences of pulses being provided in plural periods separated by "off time" when no stimulation is provided. Thus, the stimulation generating unit 110 may be configured not to output signal during an entire session but rather to only output signals during the stimulation pulses within the session.

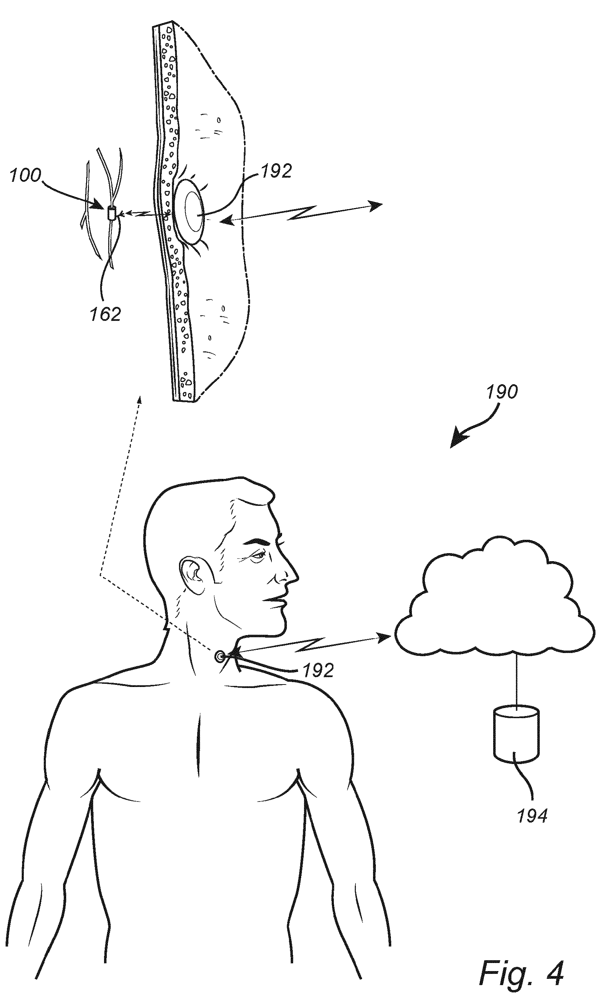

Referring now to Fig. 4, the device 100 may be incorporated in a system 190 of separate physical units.

The device 100 may be configured to be implanted in the living being, such as a human being. The first pair of electrodes 114 and the second pair of electrodes 134 are preferably configured to be in contact with the nerve 10 for providing accurate control of signals transmitted into the nerve 10 and avoiding attenuation of signals from the first pair of electrodes 114 and the second pair of electrodes 134 before reaching the nerve 10. The third pair of electrodes 144 and any additional pairs of electrodes, such as the fourth pair of electrodes 146, may also be configured to be in contact with the nerve 10.

The first stimulation generating module 112, the second stimulation generating module 132, and the third stimulation generating module 142 are preferably also implanted in the living being. This implies that wires connecting the first stimulation generating module 112 to the first pair of electrodes 114, wires connecting the second stimulation generating module 132 to the second pair of electrodes 134, and wires connecting the third stimulation generating module 142 to the third pair of electrodes 144 may be short so as to provide good quality of signals provided to the first pair of electrodes 114, the second pair of electrodes 134, and the third pair of electrodes 144.

The control unit 160 is preferably also implanted in the living being. The control unit 160 may preferably transfer control signals to the first stimulation generating module 112, the second stimulation generating module 132, and the third stimulation generating module 142 through wired communication, e.g., for accurately controlling timing of control signals. Thus, the control unit 160 may be implanted in the living being to avoid wires extending from an implant to a unit external to the body of the living being.

The control unit 160 and the stimulation generating unit 110 may be arranged within a common housing, which may be positioned close to the nerve 10 when the device 100 is implanted. The housing may provide an encasing of circuitry of the device 100 so as to protect the circuitry and to provide an appropriate outer surface for implantation in the living being.

However, in other embodiments, the third pair of electrodes 144 may be arranged externally to the living being, such as being arranged to provide the third waveform through the skin of the living being into the nerve 10. Thus, the device 100 may be partially implanted with the first stimulation generating module 112 and the second stimulation generating module 132 as well as the first pair of electrodes 114 and the second pair of electrodes 134 being implanted while the third stimulation generating module 142 and the third pair of electrodes 144 are arranged externally to the body of the living being.

The control unit 160 may be arranged in a common housing with the first and second stimulation generating modules 112, 132 or in a common housing with the third stimulation generating module 142. Alternatively, the control unit 160 may be distributed in plural modules that are commonly arranged with the first and second stimulation generating modules 112, 132 and with the third stimulation generating module 142, respectively. The plural modules may then communicate with each other for synchronizing control of the stimulation generating modules.

Hence, the device 100 may be distributed in a plurality of separate physical units forming a system.

The system 190 comprises an implant, which may incorporate the entire device 100 as described above, including the third stimulation generating module 142, the third pair of electrodes 144, and the control unit 160. The implanted device 100 may further comprise a communication unit 162, which may be configured to communicate with an external device 192 of the system 190 through wireless communication.

The implanted device 100 may thus for instance receive control signals from the external device 192 for changing parameters of the stimulation, such as parameters defining the stimulation paradigm, such as to control a location within the nerve 10 to be stimulated or a frequency, an amplitude, or a duration of a stimulation to be provided. The communication unit 162 may be provided within the control unit 160 or may be separately provided for providing wireless communication to the external device 192.

The external device 192 may be provided as a wearable for allowing use of short-range communication between the implanted device 100 and the external device 192. The external device 192 may be configured to be worn on the skin close to the implanted device 100. However, the external device 192 may alternatively be configured to be worn in any other suitable manner, such as the external device 192 being in form of a smartwatch provided with functionality for communicating with the implanted device 100. Alternatively, the external device 192 may be adapted to be arranged close to the living being, even if not being necessarily worn, such as the external device 192 being in form of a mobile phone provided with functionality for communicating with the implanted device 100.

The external device 192 may function as an intermediate device for allowing the implanted device 100 to receive and transmit information to a remote device 194. Thus, the external device 192 may enable communication between the implanted device 100 and a remote device 194 arranged anywhere, e.g., by means of communication between the external device 192 and the remote device 194 through a telecommunication or computer network, such as the Internet.

The external device 192 and/or the remote device 194 may provide information to the implanted device 100 for configuring the implanted device 100 such as for controlling parameters of the stimulation.

The implanted device 100 may further be configured to transmit information to the external device 192 and/or the remote device 194 regarding stimulations performed, which may be used as input for controlling future stimulations.

The external device 192 and the implanted device 100 may further be configured to provide wireless charging of the implanted device 100. Thus, the implanted device 100 may be wirelessly charged so as to increase lifetime of the implanted device 100.

However, in other embodiments, as discussed above, the control unit 160 and/or the third stimulation generating module 144 and the third pair of electrodes 144 may be arranged externally to the living being, such as being configured to be worn on the skin close to the implanted device 100.

In such case, the control unit 160 arranged in a housing externally to the living being may be configured communicate with the remote device 194 without any intermediate external device 192 being involved in the communication.

The device 100 may further be configured with additional functionality in a housing (incorporating the control unit 160 and/or the third stimulation generating module 142 and the third pair of electrodes 144) arranged externally to the living being. For instance, the parts of the device 100 being implanted may be wirelessly charged from a charging unit in the housing arranged externally to the living being.

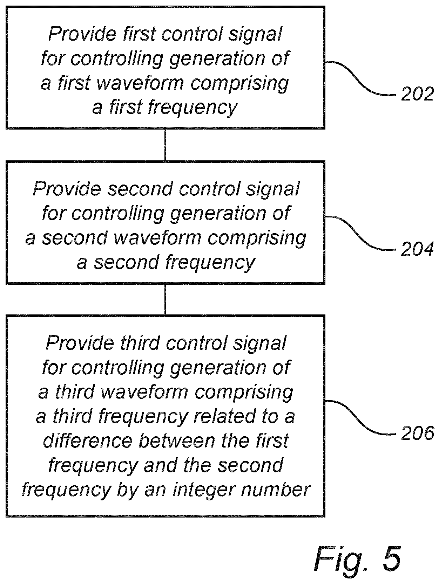

Referring now to Fig. 5, a method for controlling stimulation of a nerve 10 or a part of the nervous system will be described.

The method comprises providing 202 a first control signal for controlling generation of a first waveform comprising a first frequency to a first pair of electrodes 114 arranged in a first location in relation to the nerve 10 or a part of the nervous system.

The method further comprises providing 204 a second control signal for controlling generation of a second waveform comprising a second frequency to a second pair of electrodes 134 arranged in a second location in relation to the nerve 10 or the part of the nervous system.

The method further comprises providing 206 a third control signal for controlling generation of a third waveform comprising a third frequency to a third pair of electrodes 134 arranged in a third location in relation to the nerve 10 or the part of the nervous system.

The first control signal may be provided to a first stimulation generating module 112, the second control signal may be provided to a second stimulation generating module 132 and the third control signal may be provided to a third stimulation generating module 142. Thus, the control signals may be provided for allowing individual generation of the first waveform, the second waveform, and the third waveform, respectively, which may facilitate avoiding crosstalk between the stimulation generating modules in generation of the waveforms.

The control signals control generation of the first waveform and the second waveform with a difference between the first frequency and the second frequency defining a beat frequency and control generation of the third frequency being related to the beat frequency by an integer number.

Thus, the control signals may be provided for controlling generation of waveforms such that the third waveform may modulate interference between the first and second waveforms in the nerve 10 or part of the nervous system for allowing accurate selection of a location in the nerve 10 or the part of the nervous system in which interferential stimulation will occur.

The method may further comprise modulating the first waveform, the second waveform, and the third waveform by modulating the first frequency, the second frequency, and the third frequency, respectively, by modulating an amplitude of the first waveform, the second waveform, and the third waveform, respectively, and/or by modulating a time instant for start of the first waveform, the second waveform, and the third waveform, respectively.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. A device (100) for stimulating a nerve (10) or a part of a nervous system of a living being, said device (100) comprising:
a stimulation generating unit (110) configured to generate stimulation signals for stimulating the nerve (10) or the part of the nervous system;
a first pair of electrodes (114) configured to be arranged in a first location in relation to the nerve (10) or the part of the nervous system;
a second pair of electrodes (134) configured to be arranged in a second location in relation to the nerve (10) or the part of the nervous system;
a third pair of electrodes (144) configured to be arranged in a third location in relation to the nerve (10) or the part of the nervous system;
wherein the stimulation generating unit (110) is configured to generate a first waveform comprising a first frequency to the first pair of electrodes (114); a second waveform comprising a second frequency to the second pair of electrodes (134), and a third waveform comprising a third frequency to the third pair of electrodes (144);
wherein the first frequency and the second frequency define a beat frequency corresponding to a difference between the first frequency and the second frequency and wherein the third frequency is related to the beat frequency by an integer number,
wherein the stimulation generating unit (110) is configured to output the first waveform, the second waveform, and the third waveform for causing interferential stimulation in the nerve (10) or the part of the nervous system.

2. The device according to claim 1, wherein the device (100) is configured to be at least partially implanted in the living being with the first pair of electrodes (114), the second pair of electrodes (134), and the third pair of electrodes (144) arranged at different locations in relation to the nerve (10) or the part of the nervous system.

3. The device according to claim 2, wherein the device (100) comprises a carrier (170) configured to be arranged in relation to the nerve (10) or the part of the nervous system, wherein the first pair of electrodes (114), the second pair of electrodes (134), and the third pair of electrodes (144) are mounted in or on the carrier (170).

4. The device according to any one of the preceding claims, wherein the beat frequency is two times larger than the third frequency.

5. The device according to any one of the preceding claims, further comprising a control unit (160) configured to modulate the first waveform, the second waveform, and the third waveform by modulating the first frequency, the second frequency, and the third frequency, respectively, by modulating an amplitude of the first waveform, the second waveform, and the third waveform, respectively, and/or by modulating a time instant for start of the first waveform, the second waveform, and the third waveform, respectively, for controlling the interferential stimulation of the nerve (10) or the part of the nervous system.

6. The device according to any one of the preceding claims, further comprising at least a fourth pair of electrodes (146) configured to be arranged in a fourth location in relation to the nerve (10) or the part of the nervous system, wherein the stimulation generating unit (110) is configured to generate a fourth waveform comprising a fourth frequency to the fourth pair of electrodes (146), wherein the fourth frequency is related to the beat frequency by an integer number.

7. The device according to claim 6, wherein the stimulation generating unit (110) is configured to be controlled to selectively output the third waveform to the third pair of electrodes (144) or the fourth waveform to the fourth pair of electrodes (146) for controlling the interferential stimulation in the nerve (10) or the part of the nervous system.

8. The device according to any one of the preceding claims, wherein the stimulation generating unit (110) is configured to generate a first frequency and a second frequency, respectively, being in a range of 500 Hz - 1 MHz, such as 50 kHz - 1 MHz.

9. The device according to any one of the preceding claims, wherein the stimulation generating unit (110) is configured to generate the first frequency and the second frequency with a difference between the first frequency and the second frequency being in a range of 1 Hz - 10 kHz, such as 1 - 5 kHz.

10. The device according to any one of the preceding claims, wherein the stimulation generation unit (110) is configured to generate the first waveform, the second waveform, and the third waveform with an amplitude of the third waveform being equal to or larger than an amplitude of the first waveform and an amplitude of the second waveform.

11. The device according to any one of the preceding claims, wherein the first waveform, the second waveform, and the third waveform are sinusoidal signals.

12. The device according to any one of the preceding claims, wherein the device (100) comprises a feedback sensor (180) configured to provide feedback on stimulation of the nerve (10) or the part of the nervous system, wherein the stimulation generating unit (110) is configured to be controlled in dependence of the feedback.

13. The device according to claim 12, wherein the feedback sensor (180) is configured to monitor an effect of stimulation of the nerve (10) or the part of the nervous system by monitoring a specific biomarker.

14. A method for controlling generation of waveforms, said method comprising:
providing (202) a first control signal for controlling generation of a first waveform comprising a first frequency to a first pair of electrodes (114) arranged in a first location in relation to the nerve (10) or the part of the nervous system;
providing (204) a second control signal for controlling generation of a second waveform comprising a second frequency to a second pair of electrodes (134) arranged in a second location in relation to the nerve (10) or the part of the nervous system;
providing (206) a third control signal for controlling generation of a third waveform comprising a third frequency to a third pair of electrodes (144) arranged in a third location in relation to the nerve (10) or the part of the nervous system;
wherein the control signals control generation of the first waveform and the second waveform with a difference between the first frequency and the second frequency defining a beat frequency and control generation of the third frequency being related to the beat frequency by an integer number.

15. The method according to claim 14, further comprising modulating the first waveform, the second waveform, and the third waveform by modulating the first frequency, the second frequency, and the third frequency, respectively, by modulating an amplitude of the first waveform, the second waveform, and the third waveform, respectively, and/or by modulating a time instant for start of the first waveform, the second waveform, and the third waveform, respectively.

## Patentansprüche

1. Vorrichtung (100) zum Stimulieren eines Nervs(10) oder eines Teils eines Nervensystems eines Lebewesens, wobei die Vorrichtung (100) umfasst:
eine Stimulationsgenerierungseinheit (110), die dazu konfiguriert ist, Stimulationssignale zum Stimulieren des Nervs (10) oder des Teils des Nervensystems zu generieren;
ein erstes Paar von Elektroden (114), die dazu konfiguriert sind, an einer ersten Stelle im Verhältnis zu dem Nerv (10) oder dem Teil des Nervensystems angeordnet zu sein;
ein zweites Paar von Elektroden (134), die dazu konfiguriert sind, an einer zweiten Stelle im Verhältnis zu dem Nerv (10) oder dem Teil des Nervensystems angeordnet zu sein;
ein drittes Paar von Elektroden (144), die dazu konfiguriert sind, an einer dritten Stelle im Verhältnis zu dem Nerv (10) oder dem Teil des Nervensystems angeordnet zu sein;
wobei die Stimulationsgenerierungseinheit (110) dazu konfiguriert ist, eine erste Wellenform, die eine erste Frequenz für das erste Paar von Elektroden (114) umfasst, zu generieren; wobei eine zweite
Wellenform eine zweite Frequenz für das zweite Paar von Elektroden (134) umfasst, und eine dritte Wellenform eine dritte Frequenz für das dritte Paar von Elektroden (144) umfasst;
wobei die erste Frequenz und die zweite Frequenz eine Schwebungsfrequenz definieren, die einer Differenz zwischen der ersten Frequenz und der zweiten Frequenz entspricht, und wobei die dritte Frequenz mit der Schwebungsfrequenz über eine Ganzzahl zusammenhängt,
wobei die Stimulationsgenerierungseinheit (110) dazu konfiguriert ist, die erste Wellenform, die zweite Wellenform und die dritte Wellenform auszugeben, um eine Interferenzreizung in dem Nerv (10) oder dem Teil des Nervensystems zu verursachen.

2. Vorrichtung nach Anspruch 1, wobei die Vorrichtung (100) dazu konfiguriert ist, mindestens teilweise bei dem Lebewesen implantiert zu sein, wobei das erste Paar von Elektroden (114), das zweite Paar von Elektroden (134) und das dritte Paar von Elektroden (144) an verschiedenen Stellen im Verhältnis zu dem Nerv (10) oder dem Teil des Nervensystems angeordnet sind.

3. Vorrichtung nach Anspruch 2, wobei die Vorrichtung (100) einen Träger (170) umfasst, der dazu konfiguriert ist, im Verhältnis zu dem Nerv (10) oder dem Teil des Nervensystems angeordnet zu sein, wobei das erste Paar von Elektroden (114), das zweite Paar von Elektroden (134) und das dritte Paar von Elektroden (144) in oder auf dem Träger (170) montiert sind.

4. Vorrichtung nach einem der vorhergehenden Ansprüche, wobei die Schwebungsfrequenz doppelt so groß wie die dritte Frequenz ist.

5. Vorrichtung nach einem der vorhergehenden Ansprüche, ferner umfassend eine Steuereinheit (160), die dazu konfiguriert ist, die erste Wellenform, die zweite Wellenform und die dritte Wellenform durch Modulieren jeweils der ersten Frequenz, der zweiten Frequenz und der dritten Frequenz zu modulieren, durch Modulieren einer Amplitude jeweils der ersten Wellenform, der zweiten Wellenform und der dritten Wellenform, und/oder durch Modulieren eines Zeitpunktes zum Starten jeweils der ersten Wellenform, der zweiten Wellenform und der dritten Wellenform, um die Interferenzreizung des Nervs (10) oder des Teils des Nervensystems zu steuern, zu modulieren.

6. Vorrichtung nach einem der vorhergehenden Ansprüche, ferner umfassend mindestens ein viertes Paar von Elektroden (146), die dazu konfiguriert sind, an einer vierten Stelle im Verhältnis zu dem Nerv (10) oder dem Teil des Nervensystems angeordnet zu sein, wobei die Stimulationsgenerierungseinheit (110) dazu konfiguriert ist, eine vierte Wellenform, die eine vierte Frequenz für das vierte Paar von Elektroden (146) umfasst, zu generieren, wobei die vierte Frequenz mit der Schwebungsfrequenz über eine Ganzzahl zusammenhängt.

7. Vorrichtung nach Anspruch 6, wobei die
Stimulationsgenerierungseinheit (110) dazu konfiguriert ist, gesteuert zu werden, um selektiv die dritte Wellenform an das dritte Paar von Elektroden (144) oder die vierte Wellenform an das vierte Paar von Elektroden (146) auszugeben, um die Interferenzreizung in dem Nerv (10) oder dem Teil des Nervensystems zu steuern.

8. Vorrichtung nach einem der vorhergehenden Ansprüche, wobei die Stimulationsgenerierungseinheit (110) dazu konfiguriert ist, eine erste Frequenz und eine zweite Frequenz zu generieren, die jeweils in einem Bereich von 500 Hz bis 1 MHz, wie etwa von 50 kHz bis 1 MHz, liegen.

9. Vorrichtung nach einem der vorhergehenden Ansprüche, wobei die Stimulationsgenerierungseinheit (110) dazu konfiguriert ist, die erste Frequenz und die zweite Frequenz zu generieren, wobei eine Differenz zwischen der ersten Frequenz und der zweiten Frequenz in einem Bereich von 1 Hz bis 10 kHz, wie etwa von 1 bis 5 kHz, liegt.

10. Vorrichtung nach einem der vorhergehenden Ansprüche, wobei die Stimulationsgenerierungseinheit (110) dazu konfiguriert ist, die erste Wellenform, die zweite Wellenform und die dritte Wellenform zu generieren, wobei eine Amplitude der dritten Wellenform gleich oder größer als eine Amplitude der ersten Wellenform und eine Amplitude der zweiten Wellenform ist.

11. Vorrichtung nach einem der vorhergehenden Ansprüche, wobei die erste Wellenform, die zweite Wellenform und die dritte Wellenform sinusförmige Signale sind.

12. Vorrichtung nach einem der vorhergehenden Ansprüche, wobei die Vorrichtung (100) einen Rückmeldesensor (180) umfasst, der dazu konfiguriert ist, eine Rückmeldung über die Stimulation des Nervs (10) oder des Teils des Nervensystems bereitzustellen, wobei die Stimulationsgenerierungseinheit (110) dazu konfiguriert ist, in Abhängigkeit von der Rückmeldung gesteuert zu werden.

13. Vorrichtung nach Anspruch 12, wobei der Rückmeldesensor (180) dazu konfiguriert ist, eine Wirkung der Stimulation des Nervs (10) oder des Teils des Nervensystems durch Überwachen eines spezifischen Biomarkers zu überwachen.

14. Verfahren zum Steuern des Generierens von Wellenformen, wobei das Verfahren umfasst:
Bereitstellen (202) eines ersten Steuersignals zum Steuern des Generierens einer ersten Wellenform, die eine erste Frequenz für ein erstes Paar von Elektroden (114) umfasst, die an einer ersten Stelle im Verhältnis zu dem Nerv (10) oder dem Teil des Nervensystems angeordnet sind;
Bereitstellen (204) eines zweiten Steuersignals zum Steuern des Generierens einer zweiten Wellenform, die eine zweite Frequenz für ein zweites Paar von Elektroden (134) umfasst, die an einer zweiten Stelle im Verhältnis zu dem Nerv (10) oder dem Teil des Nervensystems angeordnet sind;
Bereitstellen (206) eines dritten Steuersignals zum Steuern des Generierens einer dritten Wellenform, die eine dritte Frequenz für ein drittes Paar von Elektroden (144) umfasst, die an einer dritten Stelle im Verhältnis zu dem Nerv (10) oder dem Teil des Nervensystems angeordnet sind;
wobei die Steuersignale das Generieren der ersten Wellenform und der zweiten Wellenform, wobei eine Differenz zwischen der ersten Frequenz und der zweiten Frequenz eine Schwebungsfrequenz definiert, und das Generieren der dritten Frequenz, die mit der Schwebungsfrequenz über eine Ganzzahl zusammenhängt, steuern.

15. Verfahren nach Anspruch 14, ferner umfassend das Modulieren der ersten Wellenform, der zweiten Wellenform und der dritten Wellenform durch Modulieren jeweils der ersten Frequenz, der zweiten Frequenz und der dritten Frequenz, durch Modulieren einer Amplitude jeweils der ersten Wellenform, der zweiten Wellenform und der dritten Wellenform, und/oder durch Modulieren eines Zeitpunktes zum Starten jeweils der ersten Wellenform, der zweiten Wellenform und der dritten Wellenform.

## Revendications

1. Dispositif (100) pour stimuler un nerf (10) ou une partie du système nerveux d'un être vivant, ledit dispositif (100) comprenant :
une unité de génération de stimulation (110) configurée pour générer des signaux de stimulation pour stimuler le nerf (10) ou la partie du système nerveux ;
une première paire d'électrodes (114) configurée pour être disposée à un premier emplacement par rapport au nerf (10) ou à la partie du système nerveux ;
une deuxième paire d'électrodes (134) configurée pour être disposée à un deuxième emplacement par rapport au nerf (10) ou à la partie du système nerveux ;
une troisième paire d'électrodes (144) configurée pour être disposée à un troisième emplacement par rapport au nerf (10) ou à la partie du système nerveux ;
dans lequel l'unité de génération de stimulation (110) est configurée pour générer une première forme d'onde comprenant une première fréquence pour la première paire d'électrodes (114) ; une deuxième forme d'onde comprenant une deuxième fréquence appliquée à la deuxième paire d'électrodes (134), et une troisième forme d'onde comprenant une troisième fréquence appliquée à la troisième paire d'électrodes (144) ;
dans lequel la première fréquence et la deuxième fréquence définissent une fréquence de battement correspondant à la différence entre la première fréquence et la deuxième fréquence et dans lequel la troisième fréquence est liée à la fréquence de battement par un nombre entier ;
dans lequel l'unité de génération de stimulation (110) est configurée pour délivrer la première forme d'onde, la deuxième forme d'onde et la troisième forme d'onde afin de provoquer une stimulation interférentielle dans le nerf (10) ou la partie du système nerveux.

2. Dispositif selon la revendication 1, dans lequel le dispositif (100) est configuré pour être au moins partiellement implanté dans l'être vivant, la première paire d'électrodes (114), la deuxième paire d'électrodes (134) et la troisième paire d'électrodes (144) étant disposées à différents emplacements par rapport au nerf (10) ou à la partie du système nerveux.

3. Dispositif selon la revendication 2, dans lequel le dispositif (100) comprend un support (170) configuré pour être disposé par rapport au nerf (10) ou à la partie du système nerveux, dans lequel la première paire d'électrodes (114), la deuxième paire d'électrodes (134) et la troisième paire d'électrodes (144) sont montées dans ou sur le support (170).

4. Dispositif selon une quelconque des revendications précédentes, dans lequel la fréquence de battement est deux fois supérieure à la troisième fréquence.

5. Dispositif selon une quelconque des revendications précédentes, comprenant en outre une unité de commande (160) configurée pour moduler la première, la deuxième et la troisième forme d'onde en modulant respectivement la première, la deuxième et la troisième fréquence, en modulant respectivement une amplitude de la première, de la deuxième et de la troisième forme d'onde, et/ou en modulant une instant de début de la première, de la deuxième et de la troisième forme d'onde, respectivement, afin de commander la stimulation interférentielle du nerf (10) ou de la partie du système nerveux.

6. Dispositif selon une quelconque des revendications précédentes, comprenant en outre au moins une quatrième paire d'électrodes (146) configurée pour être disposée à un quatrième emplacement par rapport au nerf (10) ou à la partie du système nerveux, dans lequel l'unité de génération de stimulation (110) est configurée pour générer une quatrième forme d'onde comprenant une quatrième fréquence sur la quatrième paire d'électrodes (146), dans lequel la quatrième fréquence est liée à la fréquence de battement par un nombre entier.

7. Dispositif selon la revendication 6, dans lequel l'unité de génération de stimulation (110) est configurée pour être commandée afin de délivrer sélectivement la troisième forme d'onde à la troisième paire d'électrodes (144) ou la quatrième forme d'onde à la quatrième paire d'électrodes (146) pour commander la stimulation interférentielle dans le nerf (10) ou la partie du système nerveux.

8. Dispositif selon une quelconque des revendications précédentes, dans lequel l'unité de génération de stimulation (110) est configurée pour générer respectivement une première fréquence et une deuxième fréquence, comprises entre 500 Hz et 1 MHz, par exemple entre 50 kHz et 1 MHz.

9. Dispositif selon une quelconque des revendications précédentes, dans lequel l'unité de génération de stimulation (110) est configurée pour générer la première fréquence et la deuxième fréquence, la différence entre la première et la deuxième fréquence étant comprise entre 1 Hz et 10 kHz, par exemple entre 1 et 5 kHz.

10. Dispositif selon une quelconque des revendications précédentes, dans lequel l'unité de génération de stimulation (110) est configurée pour générer la première forme d'onde, la deuxième forme d'onde et la troisième forme d'onde, une amplitude de la troisième forme d'onde étant égale ou supérieure à une amplitude de la première forme d'onde et à une amplitude de la deuxième forme d'onde.

11. Dispositif selon une quelconque des revendications précédentes, dans lequel la première forme d'onde, la deuxième forme d'onde et la troisième forme d'onde sont des signaux sinusoïdaux.

12. Dispositif selon une quelconque des revendications précédentes, dans lequel le dispositif (100) comprend un capteur de rétroaction (180) configuré pour fournir une rétroaction sur la stimulation du nerf (10) ou de la partie du système nerveux, dans lequel l'unité de génération de stimulation (110) est configurée pour être commandée en fonction de la rétroaction.

13. Dispositif selon la revendication 12, dans lequel le capteur de rétroaction (180) est configuré pour surveiller un effet de stimulation du nerf (10) ou de la partie du système nerveux en surveillant un biomarqueur spécifique.

14. Procédé de commande de génération de formes d'onde, ledit procédé comprenant :
la fourniture (202) d'un premier signal de commande pour commander la génération d'une première forme d'onde comprenant une première fréquence à une première paire d'électrodes (114) disposées à un premier emplacement par rapport au nerf (10) ou à la partie du système nerveux ;
la fourniture (204) d'un deuxième signal de commande pour commander la génération d'une deuxième forme d'onde comprenant une deuxième fréquence à une deuxième paire d'électrodes (134) disposées à un deuxième emplacement par rapport au nerf (10) ou à la partie du système nerveux ;
la fourniture (206) d'un troisième signal de commande pour commander la génération d'une troisième forme d'onde comprenant une troisième fréquence à une troisième paire d'électrodes (144) disposées à un troisième emplacement par rapport au nerf (10) ou à la partie du système nerveux ;
dans lequel les signaux de commande commandent la génération de la première forme d'onde et de la deuxième forme d'onde, une différence entre la première fréquence et la deuxième fréquence définissant une fréquence de battement, et la commande de la génération de la troisième fréquence étant liée à la fréquence de battement par un nombre entier.

15. Procédé selon la revendication 14, comprenant en outre la modulation de la première forme d'onde, de la deuxième forme d'onde et de la troisième forme d'onde par modulation de la première fréquence, de la deuxième fréquence et de la troisième fréquence, respectivement, par modulation de l'amplitude de la première forme d'onde, de la deuxième forme d'onde et de la troisième forme d'onde, respectivement, et/ou par modulation de l'instant de début de la première forme d'onde, de la deuxième forme d'onde et de la troisième forme d'onde, respectivement.
